# EUROPÄISCHE PATENTSCHRIFT

(11) **EP 3 167 287 B1**
(45) Veröffentlichungstag und Bekanntmachung des Hinweises auf die Patenterteilung: **26.08.2020**
(21) Anmeldenummer: 15744886.1
(22) Anmeldetag: 04.07.2015
(51) Int. Cl.: G01N 33/564, C07K 7/04, C07K 14/00

(54) **MARKERSEQUENZEN ZUR DIAGNOSE UND STRATIFIZIERUNG VON SYSTEMISCHE SKLEROSE PATIENTEN**
MARKER SEQUENCES FOR DIAGNOSING AND STRATIFYING SYSTEMIC SCLEROSIS PATIENTS
SÉQUENCES DE MARQUEURS POUR LE DIAGNOSTIC ET LA STRATIFICATION DE PATIENTS ATTEINTS DE SCLÉROSE SYSTÉMIQUE

(30) Priorität: 07.07.2014 EP 14176035
(43) Veröffentlichungstag der Anmeldung: 17.05.2017
(73) Patentinhaber: Oncimmune Germany GmbH, 44227 Dortmund (DE)
(72) Erfinder: BUDDE, Petra, 44229 Dortmund (DE); SCHULZ-KNAPPE, Peter, 30966 Hemmingen (DE); LÜKING, Angelika, 44892 Bochum (DE); GAMER, Martin, 89081 Ulm (DE)
(74) Vertreter: Boult Wade Tennant LLP
(86) Internationale Anmeldenummer: PCT/EP2015/065268
(87) Internationale Veröffentlichungsnummer: WO 2016/005295

(56) Entgegenhaltungen:
- WO-A2-2010/045388
- WO-A2-2015/169973
- WO-A2-2015/169973
- CATHERINE RIZOU ET AL: "B-Cell Epitope Mapping of DNA Topoisomerase I Defines Epitopes Strongly Associated with Pulmonary Fibrosis in Systemic Sclerosis", AMERICAN JOURNAL OF RESPIRATORY CELL AND MOLECULAR BIOLOGY, Bd. 22, Nr. 3, 1. März 2000 (2000-03-01), Seiten 344-351, XP055179137, ISSN: 1044-1549, DOI: 10.1165/ajrcmb.22.3.3850
- MICHAEL HECKER ET AL: "Computational analysis of high-density peptide microarray data with application from systemic sclerosis to multiple sclerosis", AUTOIMMUNITY REVIEWS, Bd. 11, Nr. 3, 18. Mai 2011 (2011-05-18), Seiten 180-190, XP028445112, ISSN: 1568-9972, DOI: 10.1016/J.AUTREV.2011.05.010 [gefunden am 2011-05-18]

## Beschreibung

Offenbart sind Verfahren zur Identifizierung von Markern für Systemische Sklerose (SSc; auch Sklerodermie oder Progressive Systemische Sklerose (PSS)) und die mit Hilfe dieses Verfahrens identifizierten Marker, die zwischen SSc und anderen Autoimmunerkrankungen einerseits und zwischen verschiedenen SSc Subgruppen andererseits differenzieren können. Weiterhin betrifft die Offenbarung Panel, Diagnostika und Test Kits, die diese Marker umfassen. Ferner offenbart sind Verfahren zum Screenen und zur Validierung von Wirkstoffen für die Anwendung bei SSc Subgruppen.

Die SSc ist eine chronische, entzündliche, rheumatische Erkrankung, die zu den klassischen immunologischen Bindegewebserkrankungen (Kollagenosen) zählt.

Die SSc ist eine heterogene Erkrankung mit übermäßiger Fibrose der Haut. Es können auch weitere Organsysteme wie z.B. Lunge, Magen-Darm, Niere, Herz und Blutgefäße betroffen sein. Zusätzlich treten Gelenksymptome (Arthritis) auf.

SSc ist eine sehr seltene Erkrankung. Die Inzidenz beträgt ca. 0,5-1,5/100.000 Einwohner/Jahr. Sie tritt meist zwischen dem 30.-50. Lebensjahr auf. Frauen sind 10-15 mal häufiger betroffen als Männer (LeRoy et al. 1988).

Klinisch werden nach LeRoy et al. (1988) zwischen der limitierten und der diffusen SSc unterschieden. In frühen Phasen der Erkrankung ist es oftmals schwierig Patienten eindeutig zu klassifizieren, welches als undifferenzierte SSc bezeichnet wird. Treten zusätzlich zur Sklerodermie auch wesentliche Symptome anderer rheumatischer Krankheiten auf, spricht man von einem Sklerodermie-Überlappungssyndrom oder Overlap-Syndrome.

Die limitierte Form der SSc tritt mit einer Frequenz von bis zu 60% auf. Diese ist charakterisiert durch eine Fibrose der Hände und Füße, die sich bis unterhalb der Ellbogen und Kniegelenke ausbreitet. Vor Auftreten der Hautfibrose besteht häufig bereits über mehrere Jahre das Raynaud-Phänomen. Häufig treten auch gastrointestinale Veränderungen (Schluckbeschwerden) und pulmonale arterielle Hypertonie (PAH) auf. Zur limitierten Form gehört auch das CREST-Syndrom: Calcinosis cutis, Raynaud-Phänomen, Ösophagusmotilitätsstörung, Sklerodaktylie und Teleangiektasie.

Bei der diffusen Form handelt es sich um die schneller und schwerwiegender verlaufende Form der SSc. Die Fibrose breitet sich dabei über die Ellbogen über den Körper und Gesicht aus. Im Gegensatz zur limitierten Form treten bereits 1-2 Jahre nach dem Auftreten des Raynaud-Phänomens Hautfibrosen auf.

Beim Sklerodermie-Überlappungssyndrom treten neben den Hauptsymptomen der Sklerodermie auch Symptome weiterer nichtorganspezifischer Autoimmunerkrankungen, wie z.B. der Myositis, Lupus Erythematodes/SLE, Sjögren-Syndrom und rheumatoide Arthritis/RA auf.

Patienten mit undifferenzierter SSc weisen das Raynaud-Syndrom auf und haben für SSc typische geschwollene Finger und pulmonale arterielle Hypertonie. Nur ein Teil der Patienten entwickelt später tatsächlich eine diffuse oder limitierte SSc.

Die Diagnose der SSc kann aufgrund des klinischen Bildes mit den typischen Hautveränderungen gestellt werden. Dies kann jedoch in Frühstadien der Erkrankung zum Teil schwierig sein. Weiterhin wird der Nachweis von antinukleären Antikörpern (ANA) herangezogen. In etwa 90% der SSc Patienten sind ANA nachweisbar. Jedoch ist der ANA-Test nicht spezifisch für SSc, da auch andere Kollagenosen und bis zu 20% der Gesunden positiv getestet werden. Die drei wichtigsten Autoantikörper bei SSc sind anti-Topoisomerase I (Scl-70), anti-Centromere (CENP) und anti-RNA-Polymerase III (anti-RNAP III). Diese Autoantikörper weisen eine hohe Spezifität für SSc auf und sind häufig mit einer Subform der SSc assoziiert. Jedoch eignen sich diese 3 Autoantikörper nur eingeschränkt zur Subtypisierung der SSc, da diese nicht ausschließlich in einem Subtyp vorkommen und deren Frequenz offenbar in verschiedenen Ethnizitäten abweichen kann. Anti-Topoisomerase-Antikörper weisen eine hohe Spezifität für SSc auf und sind in etwa 30% der Patienten mit diffuser SSc nachweisbar. Anti-Centromere Antikörper sind dagegen in etwa 50-60% der Patienten mit limitierter SSc und in 10% der Patienten mit diffuser SSc nachweisbar. Beide Autoantikörper schließen sich aus und sind nur in sehr seltenen Fällen gemeinsam in Patienten nachweisbar. Anti-RNAP III Antikörper sind häufiger in der diffusen Form nachweisbar und stellen einen Risikofaktor für die eine renale Krise dar. Insgesamt können mit den Autoantikörpern gegen anti-Topoisomerase, anti-Centromere und anti-RNAP nur etwa 70% der SSc Patienten diagnostisch identifiziert werden (Mierau et al. 2011; Mehra et al. 2013).

Seltener treten auch Antikörper gegen U1-RNP und PM-Scl Antikörper auf. Diese weisen jedoch nur eine geringe Spezifität für SSc auf: Anti-PM-Scl Antikörper werden häufig in Patienten mit Polymyositis/SSc Overlap Syndrom nachgewiesen. Antikörper gegen U1-RNP sind sowohl bei SSc als auch Mixed Connective Tissue Diseases (MCTD) und SLE nachweisbar. Bei etwa einem Drittel der Patienten werden auch Antikörper gegen typische Kollagenosen-Antigene, wie Rho52/SS-A, Ro60/SS-B, sowie citrullinated Peptide (ACPA) und Rheumafaktoren nachgewiesen.

Rizou et al. (Am J Respir Cell Mol Biol. 2000 Mar;22(3):344-51) offenbart eingige Epitope, die bei systemischer Sklerose mit Lungenfibrose assoziiert sind.

Hecker et al. (Autoimmuniuty reviews 11(3): 180-190) offenbart Computergestützte Analyse von hochdichten Peptid-Microarray-Daten mit Anwendung von systemischer Sklerose bis hin zu Multipler Sklerose.

WO2010/045388 offenbart Antikörper genen MMP-9 zur Behandlung von Systemischer Sklerose.

WO2015/169973 offenbart einige Marker für Systemische Sklerose, under anderem den Marker KDM6B.

In der klinischen Praxis ist die Diagnosestellung einer frühen Form der SSc und deren Klassifizierung in die Subgruppen diffus, limitiert oder Overlap Syndrom oftmals schwierig, da noch nicht alle Symptome vorliegen bzw. etwa 10-30% der Patienten Symptome einer anderen Kollagenose (Bindegewebserkrankung, Connective Tissue Disease) tragen. Da die verschiedenen Unterformen eine sehr unterschiedliche Prognose haben, besteht ein substantieller Bedarf an Biomarkern für eine verbesserte Diagnosestellung der SSc und für eine Einteilung in SSc Subgruppen. Weiterhin besteht ein hoher Bedarf an prognostischen und prädiktiven Biomarkern.

Ein weiteres Problem der derzeit angewendeten diagnostischen Verfahren ist, dass die Tauglichkeit der bisher untersuchten Autoantigene zur Diagnose von Organbeteiligungen und Komplikationen umstritten sind und zum Teil widersprüchliche Daten veröffentlicht wurden. Desweiteren ist es wünschenswert, im Hinblick auf die Anwendung in der individualisierten Medizin, Marker zur Verfügung zu haben, die einfach herzustellen und zu handhaben sind und dabei ein breites Feld der diagnostischen Anwendung eröffnen.

Beschrieben werden Marker, die bestimmte charakteristische Regionen von Autoantigenen erkennen, die im Zusammenhang mit SSc stehen. Bei diesen charakteristischen Regionen handelt es sich um ein oder mehrere kurze Sequenzen, die gegebenenfalls modifiziert sein können und die entweder alleine oder gemeinsam ein Motiv ausbilden, das charakteristisch für SSc und /oder eine oder mehrere SSc Subgruppen ist. Diese kurzen Marker sind einfach herzustellen und zu handhaben und eröffnen ein breites Feld der praktischen diagnostischen Anwendung im Rahmen der individualisierten Medizin.

Der Begriff Systemische Sklerose (SSc) ist beispielsweise in Pschyrembel, Klinisches Wörterbuch (Auflage 2012, DE GRUYTER) definiert.

Offenbart ist daher ein Marker zum Nachweis von Systemische Sklerose (SSc) dadurch gekennzeichnet, dass der Marker ein oder mehrere Peptidsequenzen mit einer Länge von jeweils maximal 35 oder 25 Aminosäuren umfasst, wobei beispielsweise die Peptidsequenz oder die Peptidsequenzen jeweils eine Länge von maximal 15 Aminosäuren, vorzugsweise maximal 12 oder 13 Aminosäuren aufweisen.

Gegenstand der Erfindung ist die Verwendung von einem Marker zum in vitro oder ex vivo Nachweis von Systemische Sklerose (SSc), dadurch gekennzeichnet, dass der Marker ein Peptid ist, welches aus einer Sequenz ausgewählt aus

| | |
|---|---|
| SEQ ID No. 1 | SPQPSASSSSQFSTSGGPWARERRAGEEPV, |
| SEQ ID No. 3 | SPQPSASSSSQF, |
| SEQ ID No. 4 | SSQFSTSGGPWAR, |
| SEQ ID No. 6 | GGPWARERRAGEEPV, |

oder einer Teilsequenz aus SEQ ID No: 1 besteht, wobei die Teilsequenz eine Länge von 12 oder 13 Aminosäuren hat.

In einer weiteren bevorzugten Ausfürungsform ist die Verwendung dadurch gekennzeichnet, dass der Marker in Kombination mit einem oder mehreren weitern Marker verwendet wird,
wobei der eine oder mehrere Marker unabhänging voneinander ausgewählt ist aus einem weiteren Peptid welches aus einer Sequenz ausgewählt aus SEQ ID NOs: 1-18 besteht.

| | |
|---|---|
| SEQ ID No. 1 | SPQPSASSSSQFSTSGGPWARERRAGEEPV |
| SEQ ID No. 2 | LPAPLPPSHGSS |
| SEQ ID No. 3 | SPQPSASSSSQF |
| SEQ ID No. 4 | SSQFSTSGGPWAR |
| SEQ ID No. 5 | REKLNPPTPSIYL |
| SEQ ID No. 6 | GGPWARERRAGEEPV |
| SEQ ID No. 7 | PREKLNPPTPSIYL |
| SEQ ID No. 8 | YQYQLALERYEWNEV |
| SEQ ID No. 9 | PRRRSRKPEAPRRRSPSPTPTPGPSRRGPSLGAS |
| SEQ ID No. 10 | SPSPTPTPGPSR |
| SEQ ID No. 11 | GPSRRGPSLGAS |
| SEQ ID No. 12 | TPTPGPSRRGPS |
| SEQ ID No. 13 | RSPSPTPTPGPSR |
| SEQ ID No. 14 | PRRRSRKPEAPR |
| SEQ ID No. 15 | RSPSPTPTPGPSR |
| SEQ ID No. 16 | APRRRSPSPTPTPGP |
| SEQ ID No. 17 | RRRSPSPTPTPGPSR |
| SEQ ID No. 18 | SPSPTPTPGPSRRGP, |

Offenbart sind Homologen der Sequenzen SEQ ID No. 1 bis 18 mit mindestens 95 % Homologie, Teilsequenzen von SEQ ID No. 1 bis 18, Teilsequenzen der Homologen von SEQ ID No. 1 bis 18 mit mindestens 95 % Homologie.

In einer bevorzugten Ausführungsform der Verwendung ist der Marker ein Peptid bestehend aus SEQ ID No. 3.

In einer bevorzugten Ausführungsform der Verwendung ist der Marker ein Peptid bestehend aus SEQ ID No. 4.

In einer bevorzugten Ausführungsform umfasst die Verwendung die Kombination der Marker SEQ ID No. 2 und SEQ ID No. 3.

In einer bevorzugten Ausführungsform der Verwendung besteht der Marker aus einer Teilsequenz aus SEQ ID No. 1, welche eine Länge von 12 oder 13 Aminosäuren hat.

In einer bevorzugten Ausführungsform umfasst die Verwendung die Kombination von mindestens zwei Teilsequenzen aus SEQ ID No. 1 wobei die Teilsequenzen eine Länge von 12 oder 13 Aminosäuren haben.

Die erfindungsgemässe Verwendung ist vorzugsweise zur Diagnose von SSc und/oder zur Therapieüberwachung bei SSc. Gegenstand der Erfindung ist eine Verwendung von Kombinationen gemäß von Anspruch 2, wobei mindestens drei oder vier dieser Marker verwendet werden. Die Verwendung kann zusätzlich weitere Marker oder Komponenten umfassen.

Zum Beispiel kann in der Verwendung die Kombination von SEQ ID No. 2 und SEQ ID No. 3 verwendet werden. Zum Beispiel kann in der Verwendung die Kombination von SEQ ID No. 2, SEQ ID No. 3 und SEQ ID No. 10 verwendet werden; oder die Kombination von SEQ ID No. 2, SEQ ID No. 3 und SEQ ID No. 11; ; oder die Kombination von SEQ ID No. 3, SEQ ID No. 10 und SEQ ID No. 11 oder die Kombination von SEQ ID No. 2, SEQ ID No. 3, SEQ ID No. 10 und SEQ ID No. 11. Weitere Kombinationen gemäß von Anspruch 2 kann der Fachmann in analoger Weise entsprechend zusammenstellen.

Aufgrund der hohen klinischen und serologischen Heterogenität der SSc Erkrankung ist es schwierig, mit nur einem Biomarker SSc eindeutig zu diagnostizieren. Daher ist es oft erforderlich, möglichst unkorrelierte Autoantigene zu sogenannten Paneln von Markern (Biomarker Panel für SSc) zu kombinieren. Beispielsweise im Rahmen der individualisierten Medizin können entsprechende Panel von Markern für SSc für einzelne Patienten oder Patientengruppen individuell für den betreffenden SSc Subtyp (Subgruppe) zusammengestellt werden. Ein entsprechendes Panel kann beispielsweise in Form einer Anordnung, eines Arrays oder auch eines oder mehrerer Beads ausgestaltet sein. Gegenstand der Erfindung ist somit ein fester Träger auf dem ein oder mehrere Marker fixiert sind, wobei der eine oder mehrere Marker ein Peptid ist, welches aus einer Sequenz ausgewählt aus SEQ ID No. 1 , SEQ ID No. 3, SEQ ID No. 4, oder SEQ ID No. 6 oder einer Teilsequenz aus SEQ ID No: 1 besteht, wobei die Teilsequenz eine Länge von 12 oder 13 Aminosäuren hat.

Der feste Träger kann ein Bead (Kügelchen oder Plättchen) sein.

Gegenstand der Erfindung ist eine Verwendung nach einem der Ansprüche 1-7 zur Diagnose von SSc, und/oder zur Therapieüberwachung bei SSc.

Entsprechende Verwendungen können folgende Schritte umfassen:
a. mindestens ein Marker welcher ein Peptid ist, welches aus einer Sequenz ausgewählt aus

| | |
|---|---|
| SEQ ID No. 1 | SPQPSASSSSQFSTSGGPWARERRAGEEPV, |
| SEQ ID No. 3 | SPQPSASSSSQF, |
| SEQ ID No. 4 | SSQFSTSGGPWAR, |
| SEQ ID No. 6 | GGPWARERRAGEEPV, |

oder einer Teilsequenz aus SEQ ID No: 1 besteht, wobei die Teilsequenz eine Länge von 12 oder 13 Aminosäuren hat
b. werden in vitro mit Körperflüssigkeit oder Gewebeauszug eines zu untersuchenden Individuums in Kontakt gebracht, und
c. der Nachweis einer Wechselwirkung der Körperflüssigkeit oder des Gewebeauszugs mit dem oder den Markern aus a. erfolgt.

Offenbart ist auch eine pharmazeutische Zusammensetzung zur spezifischen Anwendung bei SSc, die mindestens einen offenbarten Marker umfasst. Offenbart ist auch ein Arzneimittel zur spezifischen Anwendung bei SSc, das mindestens einen offenbarten Marker umfasst. Entsprechende (pharmazeutische) Zusammensetzungen und Arzneimittel können weitere Zusatz- und/ oder Hilfsstoffe umfassen.

Offenbart ist auch ein Antikörper zur spezifischen Anwendung zur Behandlung von SSc. Offenbart ist ein Antikörper zur spezifischen Anwendung zur Behandlung von SSc, umfassend mindestens eine offenbarte Bindungsregion oder ein offenbartes Epitop. Offenbart ist ein Antikörper zur spezifischen Anwendung zur Behandlung von SSc, umfassend mindestens ein offenbartes Epitop. Offenbart ist ein Antikörper zur spezifischen Anwendung zur Behandlung von SSc, umfassend mindestens eine Peptidsequenz SEQ ID No. 1 bis 18; oder mindestens ein Homologes der Peptidsequenzen SEQ ID No. 1 bis 18 mit mindestens 95 % Homologie; odermindestens eine Teilsequenz von SEQ ID No. 1 bis 18; oder mindestens eine Teilsequenz der Homologen von SEQ ID No. 1 bis 18 mit mindestens 95 % Homologie.

Offenbart ist auch ein Target zur Therapie von SSc ausgewählt aus den offenbarten Markern, beispielsweise eine Bindungsregion oder ein Epitop, beispielsweise ausgewählt aus den Sequenzen SEQ ID No. 1 bis 18, den Homologen der Sequenzen SEQ ID No. 1 bis 18 mit mindestens 95 % Homologie, den Teilsequenzen von SEQ ID No. 1 bis 18 und den Teilsequenzen der Homologen von SEQ ID No. 1 bis 18 mit mindestens 95 % Homologie sowie der für die Sequenzen SEQ ID No. 1 bis 18, deren Homologe, Teilsequenzen und Homolge der Teilsequenzen kodierenden Nukeinsäuren.

Offenbart ist ein Arzneimittel oder einen Wirkstoff oder eine Prodrug für SSc entwickelt und erhältlich durch den Einsatz eines offenbarten SSc Markers.

Offenbart ist eine Affinitätsmaterial, dass einen oder mehrere offenbarte Marker umfasst. Das Affinitätsmaterial kann beispielsweise zur Durchführung einer Apherese oder Blutwäsche für Patienten mit SSc verwendet werden. Offenbart ist somit die Verwendung der offenbarten Marker, vorzugsweise in Form einer Anordnung, als Affinitätsmaterial zur Durchführung einer Blutwäsche im weiteren Sinne, wobei Substanzen aus Körperflüssigkeiten eines Patienten mit SSc, wie Blut oder Plasma, an die offenbarten Marker binden und folglich der Körperflüssigkeit selektiv entzogen werden können.

Offenbart ist auch ein Verfahren zum Screening von Wirkstoffen zur Anwendung bei SSc, wobei
a. mindestens ein offenbarter Marker, beispielsweise eine Bindungsregion oder ein Epitop oder ein offenbartes Panel oder ein offenbarter Antikörper
b. mit einer zu untersuchenden Substanz in Kontakt gebracht wird und
c. der Nachweis einer Wechselwirkung der zu untersuchenden Substanz mit dem oder den Markern, dem Panel oder Antikörpern aus a. erfolgt.

Die Verwendung gemäß einer der Anspüche 1-8 kann zusammen mit der Verwendung bereits bekannter und / oder neuen Wirkstoffen (begleitende Diagnostik, Companion Diagnostik) erfolgen. Offenbart ist die Verwendung eines oder mehrerer offenbarter Marker zur Definition von Patientengruppen in klinischen Studien, beispielsweise dSSc, lSSc oder SSc-OS Subgruppen für Medikamentenstudien zu rekrutieren. Offenbart ist die Verwendung eines oder mehrerer offenbarter Marker zur Identifizierung von spezifischen Antikörpersignaturen in SSc-Patienten-Subgruppen. Offenbart ist die Verwendung eines oder mehrerer offenbarter Marker zur individualisierten Diagnostik und / oder Therapie bei einzelnen Patienten, Patientengruppen, Kohorten, Bevölkerungsgruppen, Varianten von SSc, Stadien von SSc. Offenbart ist die Verwendung eines oder mehrerer offenbarter Marker zur Analyse von Autoantikörperprofilen von Patienten, insbesondere zur qualitativen und/oder quantitativen Analyse von Autoantikörpern und/oder zur Überwachung von Veränderungen von Autoantikörperprofilen, die mit SSc oder SSc Subgruppen assoziiert sind, beispielsweise in Körperflüssigkeiten wie Serum, Gewebe oder Gewebeauszügen des Patienten. Gemäß der Verwendung nach einem der Ansprüche 1-7 kann der Nachweis einer Wechselwirkung der Körperflüssigkeit oder dem Gewebeauszugs und dem oder den Markern erfolgen und dadurch ein SSc assoziiertes Autoantikörperprofil des Patienten oder einer Kohorte oder einer Bevölkerungsgruppe abgebildet werden. Offenbart ist die Verwendung eines oder mehrerer offenbarter Marker, wobei der Nachweis einer Wechselwirkung der Körperflüssigkeit oder des Gewebeauszugs mit dem oder den offenbarten Markern die Vorhersage eines Krankheitsverlaufs erlaubt (Prognose). Offenbart ist die Verwendung eines oder mehrerer offenbarter Marker, wobei der Nachweis einer Wechselwirkung der Körperflüssigkeit oder des Gewebeauszugs mit dem oder den ofenbarten Markern eine Vorhersage bezügliche eines Ansprechens oder Nichtansprechens auf eine Therapie oder einen Wirkstoff ermöglicht (z.B Therapiesteuerung, Wikstoffauswahl). Die Verwendung eines oder mehrerer offenbarter Marker ermöglicht die Analyse von Autoantikörperprofilen von Patienten, insbesondere die quantitative Analyse und/oder die Überwachung von Veränderungen von Autoantikörperprofilen von SSc Patienten. Der Nachweis einer Wechselwirkung der Körperflüssigkeit oder des Gewebeauszugs mit der oder den SSc Markern kann beispielsweise durch eine Sonde, insbesondere durch einen Antikörper erfolgen.

In einer bevorzugten Ausführungsform werden mindestens 2, beispielsweise 3, 4, 5, 6, 7, 8, 9, 10 oder mehr Marker zusammen oder in Kombination verwendet, entweder gleichzeitig oder nacheinander, gemäss von Anspruch 2.

Gemäss nach Anspruch 9 umfasst die Erfindung die in Anspruch 9 genannten Marker auf einem festen Träger, beispielsweise einem Filter, einer Membran, einem kleinen Plättchen oder Kügelchen, beispielsweise einem magnetischen oder Fluorophor-markierten Kügelchen, einem Silizium-Wafer, einem Bead, einem Chip, einem massenspektrometrometrischen Target oder einer Matrix oder ähnlichem. Als Träger sind unterschiedliche Materialien geeignet, die dem Fachmann bekannt sind, beispielsweise Glas, Metall, Kunststoff, Filter, PVDF, Nitrocellulose oder Nylon (z.B. Immobilon P Millipore, Protran Whatman, Hybond N+ Amersham).

Der Träger kann beispielsweise einem Gitter entsprechen, dass die Größenordnung einer Mikrotiterplatte (8-12 Wells Streifen, 96 Wells, 384 Wells oder mehr), eines Silizium-Wafers, eines Chips, eines massenspektrometrischen Targets oder einer Matrix besitzt. In einer Ausführungsform des Trägers kann der jeweilige SSc Marker in unterschiedlichen Mengen in einem oder mehreren Bereichen des Trägers repräsentiert sein. Dies erlaubt eine Variation der Sensitivität. Die Bereiche können jeweils eine Gesamtheit von SSc Markern aufweisen, d.h. eine genügende Zahl an SSc Markern, insbesondere 2, 3, 4, 5, 6, 7, 8, 9 oder 10 oder mehr, 20 bis 50 (numerisch) oder mehr, bevorzugt mehr als 100, besonders bevorzugt 150 oder mehr, beispielsweise 25000 oder 5000 oder 10000. Dabei können gleiche Marker und / oder verschiedene Marker verwendet werden.

Ein "Proteinarray" im Sinne dieser Erfindung ist die systematische Anordnung von SSc Markern auf einem festen Träger und wobei der Träger jede beliebige Form und/oder Größe haben kann und wobei der Träger ein fester Träger ist.

Gemäss nach Anspruch 9 sind die SSc Marker auf dem Träger fixiert, vorzugsweise gespottet oder immobilisiert, aufgedruckt oder ähnliches, insbesondere reproduzierbar aufgebracht. Ein oder mehrere SSc Marker können mehrfach in der Gesamtheit aller SSc Marker präsent sein und in unterschiedlichen Mengen bezogen auf einen Spot vorliegen. Ferner können die SSc Marker auf dem Träger standardisiert sein (z.B. mittels serieller Verdünnungsreihen von z.B. Humanglobulinen als interne Kalibratoren zur Datennormalisierung und quantitativen Auswertung).

Gegebenenfalls kann auch ein Standard (z.B. ein Gold Standard) auf dem Träger aufgebracht sein. In einer weiteren Ausführungsform liegt bzw. lieger der bzw. die SSc Marker als Klonsequenzen bzw. Klone vor.

Die Marker können bei den in dieser Anmeldung benannten Verwendungen mit bekannten Biomarkern für SSc oder Biomarkern für andere Erkrankungen kombiniert, ergänzt oder erweitert werden. Vorzugsweise sind bei einer solchen Kombination mindestens 50 %, vorzugsweise 60 %, besonders bevorzugt 70 % oder mehr Marker umfasst.

Es erfolgt die Verwendung der SSc Marker außerhalb des menschlichen oder tierischen Körpers, daher erfolgt die Diagnose ex vivo / in vitro.

"Diagnose" im Sinne dieser Erfindung bedeutet die positive Feststellung von SSc mit Hilfe der Marker und die Zuordnung der Patienten bzw. deren Symptomen zu der Erkrankung SSc dar. Der Begriff der Diagnose umfasst die medizinische Diagnostik und diesbezügliche Untersuchungen, insbesondere die in-vitro Diagnostik und Labordiagnostik, ebenfalls Proteomics und Nukleinsäureblots. Weitere Untersuchungen können zur Absicherung und zum Ausschluss anderer Krankheiten vonnöten sein. Daher umfasst der Begriff Diagnose insbesondere die Differentialdiagnose von SSc mittels der Marker.

"Stratifizieren (auch: Stratifikation) oder Therapiesteuerung" bedeutet, dass beispielsweise die Verfahren Entscheidungen zur Behandlung und Therapie des Patienten erlauben, sei es Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf bzw. Ätiologie oder Klassifizierung einer Erkrankung, z.B. in einen neuen oder bestehenden Subtyp oder die Differenzierung von Krankheiten und dessen Patienten. Es umfasst der Begriff "Stratifizierung" insbesondere die Risikostratifizierung mit der Prognose eines "outcome" eines nachteiligen gesundheitlichen Ereignisses. Ferner umfasst ist die Stratifizierung der Patienten mit SSc in neue oder etablierte SSc Subgruppen sowie die sinnvolle Auswahl von Patientengruppen für die klinische Entwicklung von neuen Therapeutika. Der Begriff Therapiesteuerung umfasst ebenfalls die Einteilung von Patienten in Responder und Nicht-Responder bezüglich einer Therapie oder dessen Therapieverlauf.

Es bedeutet "Therapiesteuerung", beispielsweise Vorhersage und Überwachung des Ansprechens auf ein Arzneimittel oder eine Therapie sowie die Nachsorge.

"Prognose" bedeutet die Vorhersage des Krankheitsverlaufs.

Im Rahmen dieser Erfindung wird unter "Patient" bzw. "Patientin" ein beliebiger Proband, ein beliebiges Individuum - Mensch oder Säugetier - verstanden, mit der Maßgabe, dass der Proband bzw. das Individuum auf SSc untersucht wird.

Marker sind Aminosäuresequenzen, welche aus einer Sequenz ausgewählt aus

| | |
|---|---|
| SEQ ID No. 1 | SPQPSASSSSQFSTSGGPWARERRAGEEPV, |
| SEQ ID No. 3 | SPQPSASSSSQF, |
| SEQ ID No. 4 | SSQFSTSGGPWAR, |
| SEQ ID No. 6 | GGPWARERRAGEEPV, |

oder einer Teilsequenz aus SEQ ID No: 1 bestehen,
wobei die Teilsequenz eine Länge von 12 oder 13 Aminosäuren hat. In einigen Auführungsformen werden Aminosäuresequenzen gemäß der Definition im anliegenden Sequenzprotokoll (SEQ ID No. 1 bis SEQ ID No. 18) verwendet. Der Marker kann eine Wechselwirkung mit SSc-spezifischen Substanzen aus der Körperflüssigkeit oder Gewebeauszug eines Patienten mit SSc ausbilden (z.B. Antigen (Epitop) / Antikörper (Paratop) Wechselwirkung). Die SSc-spezifischen Substanzen aus der Körperflüssigkeit oder Gewebeauszug treten entweder nur oder zumindest verstärkt bei SSc auf beziehungsweise werden exprimiert, wohingegen diese Substanzen bei Patienten ohne SSc oder Gesunden nicht oder zumindest in geringerem Maße (geringere Menge, geringerer Konzentration) vorhanden sind. Die Marker für SSc zeichnen sich andererseits dadurch aus, dass sie eine Wechselwirkung mit Substanzen aus der Körperflüssigkeit oder Gewebeauszug von Patienten mit SSc eingehen, weil bestimmte Substanzen nicht mehr oder zumindest in deutlich geringerer Menge/Konzentration bei SSs auftreten beziehungsweise exprimiert werden, wohingegen diese Substanzen bei Patienten ohne SSs vorhanden oder zumindest in deutlich höherem Maße vorhanden sind. Marker für SSc können auch in gesunden Probanden vorhanden sein, jedoch verändert sich ihre Menge (Konzentration) beispielsweise bei der Entstehung, Etablierung und Therapie von SSc. Ein oder mehrere Marker können auf diese Weise ein Profil von Substanzen aus Körperflüssigkeit und Gewebeauszug abbilden, beispielsweise ein SSc assoziiertes Autoantikörperprofil des betreffenden Patienten. Erfindungsgemäße Marker sind Biomarker für SSc.

In einer besonders bevorzugten Ausführungsform erkennt und / oder bindet der erfindungsgemäße Marker an Autoantikörper, die im Verlauf der Entstehung, Etablierung und Therapie von SSc (verstärkt) vorhanden sind oder in geringerem Maße (oder nicht mehr) vorhanden sind. Im Rahmen der vorliegenden Erfindung werden insbesondere die Autoantikörper detektiert, die beim Auftreten und im Laufe der Entstehung von SSc gebildet werden und / oder in ihrer Expression hoch- beziehungsweise herunterreguliert werden. Diese Autoantikörper können mit Hilfe der beschriebenen Verfahren und Marker nachgewiesen werden und ihr Nachweis und ihre Überwachung (z.B. der Menge) kann zur Früherkennung, Diagnose und / oder Therapieüberwachung / Therapiesteuerung sowie zur Prognose und Vorhersage des Risikos des Wiederauftretens von SSc im Rahmen der Nachsorge verwendet werden.

Die Autoantikörperprofile können bereits bei Verwendung eines einzigen SSc Markers ausreichend charakterisiert werden. In anderen Fällen werden zwei oder mehr SSc Marker notwendig sein, um ein Autoantikörperprofil abzubilden, das für SSc spezifisch ist. Die Anzahl der verwendeten Marker kann sich beispielsweise im Rahmen der Verwendung in Diagnostika unterscheiden. Während Schnell- bzw. Routinetests, die der Patient zu Hause durchführt, nur ein, 2, 3 oder 4 (wenige) Marker umfasst, kann ein hochsensitiver diagnostischer Test mehrere bis viele, z.B. 5-20 oder mehr Marker umfassen.

Es können Autoantikörper mit SSc Markern nachgewiesen werden. Es können diese Autoantikörper mit SSc Markern nachgewiesen werden, die sich von dem gleichen Individuum ableiten. Autoantikörper können bereits viele Jahre vor Auftreten der ersten Krankheitssymptome vom Patienten gebildet werden. Damit wäre eine Früherkennung, Diagnose und auch Prognose und vorbeugende Behandlung bzw. Umstellung der Lebensweise und andere Möglichkeiten der Prävention bereits Jahre vor dem sichtbaren Krankheitsausbruch möglich. Die erfindungsgemäßen Verwendungen ermöglichen somit ein im Vergleich zu bekannten Verfahren sehr frühes Eingreifen, was die Prävention, Behandlungsmöglichkeiten und Folgen von SSc deutlich verbessert.

Da sich die SSc-assoziierten Autoantikörperprofile während der Etablierung und Behandlung/Therapie von SSc verändern, ermöglicht die Erfindung auch den Nachweis und die Überwachung von SSc in jedem Stadium der Entstehung und Behandlung sowie die Überwachung im Rahmen der SSc Nachsorge. Die offenbarten Mittel, beispielsweise eine entsprechendes Diagnostikum oder ein Test Kit erlauben auch eine einfache Handhabung zu Hause durch den Patienten und die kostengünstige routinemäßige Vorsorge zur Früherkennung.

Der Nachweis von SSc assoziierten Autoantikörpern beispielsweise im Serum oder Plasma von Patienten hat gegenüber anderen Biomarkern den Vorteil einer hohen Stabilität und Lagerfähigkeit und einer guten Nachweisbarkeit. Auch unterliegt die Anwesenheit von Autoantikörpern keinem circardianen Rhythmus, so dass die Probennahme unabhängig von Tageszeit, Nahrungsaufnahme und dergleichen ist.

Gegenstand der Erfindung ist die Verwendung eines oder mehrerer Marker gemäß der Ansprüche Nachweis in einer Körperflüssigkeit, insbesondere Blut, Vollblut, Blutplasma, Blutserum, Patientenserum, Urin, Cerebrospinalflüssigkeit, Synovialflüssigkeit oder eines Gewebeauszuges des Patienten. Die Erfindung betrifft insbesondere die Verwendung der Marker an diesen Körperflüssigkeiten und Gewebeauszügen zur Diagnose. Diese Aufzählung ist jedoch nicht abschließend, so ist beispielsweise auch die kopplung mit DNA-Tests möglich.

Offenbart sind Homologe der Marker SEQ ID NO. 1 bis 18. Homologe sind zum einen solche mit Homologie der Amino- bzw. Nukleinsäuresequenz (Sequenz-Homologe) und solche, bei denen die entsprechende Sequenz modifiziert ist, beispielsweise die Proteinvarianten, die zwar die gleiche Aminosäuresequenz aufweisen, sich aber im Hinblick auf die Modifikation, insbesondere die posttranslationale Modifikation, unterscheiden. Offenbart sind Modifikationen der Nukleinsäuresequenz und der Aminosäuresequenz, beispielsweise Citrullinierung, Acetylierung, Phosphorylierung, Glykosilierung, Methylierung, polyA-Strang-Verlängerung und andere dem Fachmann einschlägig bekannte Modifikationen.

Teilsequenzen sind solche Aminosäuresequenzen, die gegenüber der vollständigen Sequernz SEQ ID No. 1 bis 18 verkürzt sind. Die Deletion kann sich dabei an dem oder den Ende und/oder innerhalb der Peptidsequenz befinden. Offenbart sind beispielsweise Teilsequenzen, die 1, 2, 3, 4 oder 5 Aminosäuren weniger aufweisen, als die Sequenzen SEQ ID No. 1 bis 18. Offenbart sind auch Marker, die sich von den Sequenzen SEQ ID No. 1 bis 18 dadurch unterscheiden, dass sie ein oder mehrere Insertionen beinhalten, wobei die Insertionen beispielsweise 1 bis 10 oder mehr Aminosäuren, beispielsweise 1, 2, 3, 4 oder 5 Aminosäuren lang sind und die Sequenzen aber ansonsten identisch oder homolog zu den Sequenzen SEQ ID No. 1 bis 18 sind. Offenbart sind Teilsequenzen, die mindestens 95 %, vorzugsweise mindestens 97 %, besonders bevorzugt mindestens 98 % oder 99 % der Länge der Marker mit den Sequenzen SEQ ID No. 1 bis 18 aufweisen.

Zusätzlich können die SSc Marker in der jeweiligen Form als Fusionsprotein vorliegen, welches beispielsweise mindestens ein Affinitätsepitop oder "Tag" enthält, wobei das Tag beispielsweise ausgewählt wird aus c-myc, His-Tag, Arg-Tag, FLAG, alkalische Phosphatase, V5-Tag, T7-Tag oder Strep-Tag, HAT-Tag, NusA, S-Tag, SBP-Tag, Thioredoxin, DsbA oder das Fusionsprotein beispielsweise ein oder mehrere zusätzliche Domänen aufweist, beispielsweise eine Cellulose-bindende Domäne, grünfluoreszierendes Protein, Maltose bindendes Protein, Calmodulin-bindendes Protein, Glutathione S-transferase oder lacZ.

Offenbart ist ein Assay, beispielsweise ein multiplex Assay, ein Bead basierter Assay oder Proteinarray zum Identifizieren und Charakterisieren einer Substanz, beispielsweise eines Hits, einer Leitsubstanz, eines Wirkstoffs für SSc. Dabei wird eine zu untersuchende Substanz eingesetzt. Diese kann ein beliebiges natives oder nicht-natives Biomolekül, ein (synthetisches) chemisches Molekül, ein Naturstoff, eine Mischung oder eine Substanzbibliothek sein. Nachdem die zu untersuchende Substanz einen SSc Marker kontaktiert hat, erfolgt die Auswertung des Bindungserfolges, beispielsweise unter Verwendung mit handelsüblicher Image-Analyse Software (GenePix Pro (Axon Laboratories), Aida (Raytest), ScanArray (Packard Bioscience).

Die Visualisierung von Bindungen, Bindungserfolge, Wechselwirkungen wie Protein-Protein-Wechselwirkungen (z.B. Protein an SSc Marker wie Antigen/Antikörper) oder entsprechende "Mittel zum Nachweis des Bindungserfolges" kann beispielsweise mittels Fluoresenzmarkierung, Biotiniylierung, Radio-Isotopen-Markierung oder kolloidale Gold- oder Latex-Partikel-Markierung in üblicher Weise erfolgen. Ein Nachweis von gebundenen Antikörpern erfolgt vorzugsweise mit Hilfe von sekundären Antikörpern, die mit handelsüblichen Reportermolekülen markiert sind (z.B. Cy-, Alexa-, Dyomics, FITC- oder ähnliche Fluoreszenzfarbstoffe, kolloidale Gold- oder Latex-Partikel), oder mit Reporter-Enzymen wie alkalischer Phosphatase, Meerrettichperoxidase, usw. und den entsprechenden colorimetrischen, fluoreszenten oder chemolumineszenten Substraten. Eine Auslesung erfolgt z.B. visuell.

Die nachfolgenden Beispiele dienen der Erläuterung der Erfindung ohne die Erfindung jedoch auf die Beispiele einzuschränken. In den nachfolgen Figuren wird die Systemische Sklerose mit PPS (Progressive Systemische Sklerose) bezeichnet.
Figur 1: Die Intensitätsplots der CENPA Peptide, die mit den Patientenproben PG520-P01-2012-076, PG520-P01-2012-090, PG520-P01-2012-100 und PG520-P01-2012-138 bei einer Verdünnung von 1:5000 erzielt wurden.
Figur 2: Die Intensitätsplots der CENPA Peptide, die mit der Patientenprobe PG520-P01-2012-076 erzielt wurden.
Figur 3: Die Intensitätsplots der CENPA Peptide, die mit der Patientenprobe PG520-P01-2012-090 erzielt wurden.
Figur 4: Die Intensitätsplots der CENPA Peptide, die mit der Patientenprobe PG520-P01-2012-100 erzielt wurden.
Figur 5: Die Intensitätsplots der CENPA Peptide, die mit der Patientenprobe PG520-P01-2012-138 erzielt wurden.
Figur 6: Die Intensitätsplots der KDM6B Peptide, die mit den Patientenproben PG520-P01-2012-076, PG520-P01-2012-090, PG520-P01-2012-100 und PG520-P01-2012-138 bei einer Verdünnung von 1:5000 erzielt wurden.
Figur 7: Die Intensitätsplots der KDM6B Peptide, die mit der Patientenprobe PG520-P01-2012-138 erzielt wurden.

### Beispiele

### Beispiel 1: Beschreibung und Ziel der Studie

Das Ziel der Studie bestand darin, das relevante Epitop / die relevanten Epitope des neuartigen Autoantigens KDM6B zu bestimmen. Im Rahmen dieser Studie wurden Peptid Mikro-Arrays von PEPperPRINT(http://www.PEPperPRINT.com) eingesetzt.

Das CENPA Antigen wurde als Zielantigen analysiert und für die Bewertung der Technologie verwendet. Darüber hinaus wurde das neue KDM6B Antigen als Antigen für die Diagnose von SSc getestet. Obwohl dieses Antigen ursprünglich einen sehr großen Umfang hat, wurde es nur als ein kurzes N-Terminal Fragment exprimiert.

Für die Epitopkartierung wurden 15mer Peptide generiert, mit einer Überlappung von Peptid zu Peptid von 14 Aminosäuren.

Alle Peptide, welche die Sequenz von beiden Antigenen decken, wurden mittels PEPperPRINT auf einem flachen Mikro-Array gedruckt. Insgesamt wurden vier Mikro-Arrays generiert. Zur Analyse wurden drei Seren von SSc Patienten, welche Reaktivität auf beide Antigene zeigen, selektiert und auf den Arrays inkubiert. Ein Serum eines SSc Patienten, das keine Reaktivität auf diese beiden Antigene zeigte, wurde als Negativkontrolle verwendet.

Das primäre Ziel der vorliegenden Studie bestand darin, die relevanten immunogenen Regionen / Epitope von CENPA, markiert durch SSc Seren, mittels Peptid Mikro-Arrays zu identifizieren und die Resultate mit dem bereits veröffentlichten Wissensstand zu vergleichen.

Das sekundäre Ziel der vorliegenden Studie bestand darin, putative immunogene Epitope eines neuartigen Antigens (KDM6B) zu identifizieren.

Das dritte Ziel bestand darin, zu untersuchen, ob sich die drei Krankheitsseren gleichartig verhalten, oder ob sie eine biologische Diversität aufgrund von polyklonaler Immunreaktion zeigen.

### Beispiel 2: Auswahl der SSc Patienten- und Kontrollproben

Es wurden insgesamt vier verschiedene Proben von SSc Patienten auf der Grundlage ihrer Reaktivität auf die jeweiligen Antigene CENPA und KDM6B (Tabelle 1) ausgewählt.

Drei der SSc Patientenproben (PG520-P01-2012-076, PG520-P01-2012-090, PG520-P01-2012-100), die positiv für anti-CENPA und anti-KDM6B Proteinfragmente getestet wurden, wurden als Positivproben ausgewählt.

Eine SSc Kontrollprobe (PG520-P01-2012-138), welche negative Testergebnisse für anti-CENPA und anti-KDM6B zeigte, wurde inkludiert. Das schließt jedoch nicht die Möglichkeit aus, dass diese Probe eventuell negativ auf ein Epitop reagiert, das sich in Entfernung von der Region befindet, welche in dem vorigen Screen getestet wurde.

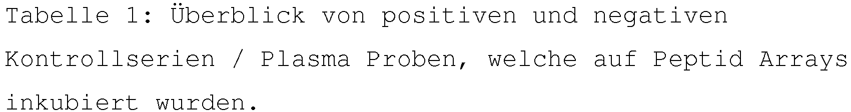

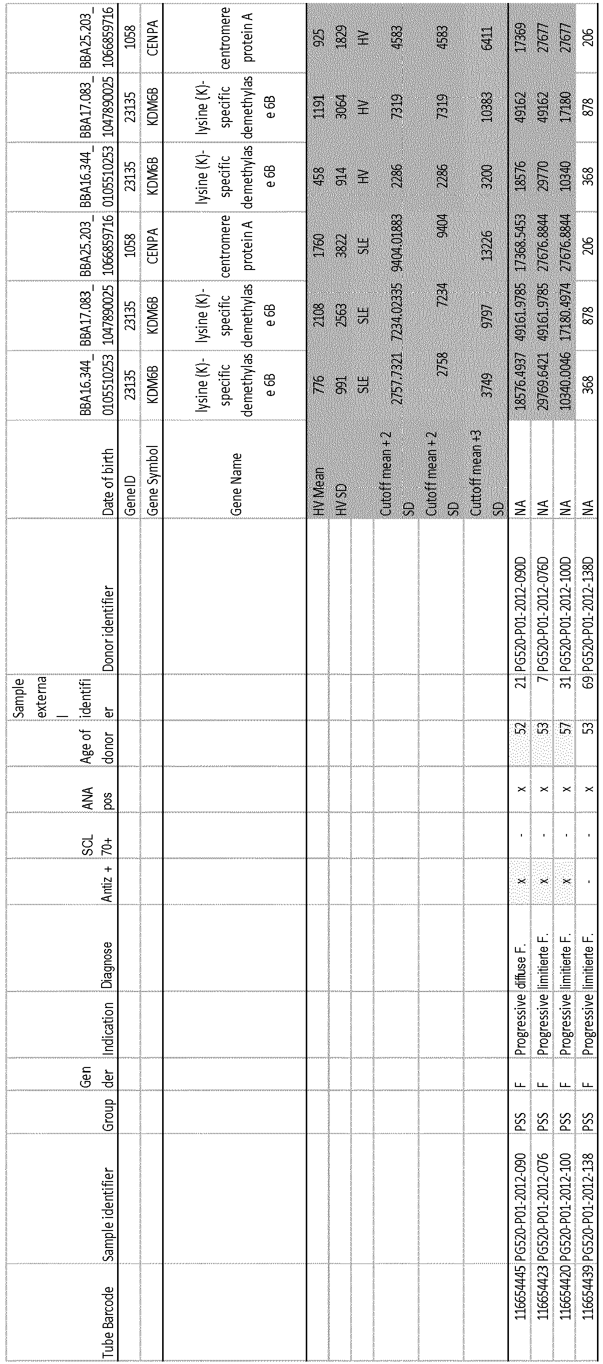

### Beispiel 3: Epitopkartierung mittels Peptid-Mikro-Arrays

### 3.1 Beschreibung von Peptid Mikro-Arrays

Die Antigene CENPA und KDM6B wurden in 15mer Peptide umgesetzt mit einer Überlappung zwischen Peptiden von 14 Aminosäuren, was zu 1.831 verschiedenen Peptiden, in zweifacher Ausführung gedruckt (insgesamt 3.662 Peptidspots) führte. Die entsprechenden Peptid Mikro-Arrays wurden zusätzlich durch Flag und HA Kontrollpeptide (je 124 Spots) gerahmt.

### 3.2 Experimentelle Bedingungen und Vorgehensweise

Die experimentellen Bedingungen wurden wie durch PEPperPRINT angezeigt, aufgelistet:
Inkubationspuffer: PBS, pH 7,4 mit 0,05% Tween 20 und 10% Rockland Blockierungspuffer
Waschpuffer: PBS,pH 7,4 mit 0,05% Tween 20 (2x1 Min nach jedem Assay)
Blockierungspuffer: Rockland Blockierungspuffer MB-070 (60 Min vor dem ersten Assay)
Bedingungen für das Assay: Serumverdünnungen von 1:5000 und 1:1000 im Inkubationspuffer; Inkubation während 16 h bei 4°C und Schütteln bei 500 Rpm.
Sekundärer Antikörper: F(ab')2 Ziege anti-human IgG(H+L) konj. DyLight680; 30 Min Anfärben bei RT und einer Verdünnung von 1:5000
Kontrollantikörper: Monoklonales anti-HA (12CA5)-DyLight680, monoklonales anti-FLAG(M2)-DyLight800; Anfärben im Inkubationspuffer während 1 h bei RT und einer Verdünnung von 1:1000
Scanner: LI-COR Odyssey Imaging System; Scanning Offset 1 mm, Resolution 21 pm, Scanning Intensität grün/rot von 7/7
Mikroarray Daten: MicroarrayData_PG520-P01-2012-076.xlsx, MicroarrayData_PG520-P01-2012-090.xlsx, MicroarrayData_PG520-P01-2012-100.xlsx, MicroarrayData_PG520-P01-2012-138.xlsx, MicroarrayData_Summary.xlsx
Mikroarray Identifikation: 000616_02, 000616_03, 000646_05, 000646_06 (zwei Arraykopien pro Mikroarray)

Die Vorfärbung von einem der Peptid Arrays wurde mit dem F(ab')2 Ziegen anti-humanem IgG(H+L) konj. DyLight680 Antikörper bei einer Verdünnung von 1:5000 durchgeführt, um die Hintergrundinteraktionen mit den Peptiden, die keine Antigene aufwiesen, zu untersuchen, welche die Haupt-Assays beeinträchtigten könnten. Die darauf folgende Inkubation der Peptid Mikroarrays mit den Humanseren PG520-P01-2012-076, PG520-P01-2012-090, PG520-P01-2012-100 und PG520-P01-2012-138 bei Verdünnungen von 1:5000 und 1:1000 im Inkubationspuffer wurde von Anfärben mit dem sekundären Antikörper sowie Readout bei einer Scanning Intensität von 7 (rot) gefolgt. HA und Flag Kontrollpeptide, welche die Peptid Arrays rahmen, wurden schließlich angefärbt als interne Qualitätskontrolle, um die Qualität des Assay, sowie die Integrität des Peptid Mikroarrays zu bestätigen (Scanning Intensitäten rot/grün 7/7) .

### Beispiel 4: Datenanalyse

Die Datenanalyse wurde mittels PEPperPRINT wie nachstehend durchgeführt. Die Quantifizierung der Spotintensitäten und die Peptid Erfassung wurden mithilfe des PepSlide® Analysators durchgeführt. Ein Softwarealgorithmus gliedert die Fluoreszenzintensitäten eines jeden Spots in Roh-, Vordergrund- und Hintergrundsignal auf und kalkuliert die Standardabweichung von mittleren Vordergrundintensitäten. Auf der Grundlage von durchschnittsberechneten mittleren Vordergrundintensitäten wurden Intensitätskarten generiert, und die Binder wurden in den Peptidkarten durch einen Intensitätsfarbcode rot für hohe Spotintensitäten und weiß für niedrige Spotintensitäten markiert.

PEPperPRINT zeichnete zusätzlich durchschnittsberechnete Spotintensitäten von allen Assays gegen die gelinkten Antigensequenzen von dem N-Terminus von CENPA zu dem C-Terminus von KDMB6 auf, um die gesamten Spotintensitäten sowie das Verhältnis zwischen Signal und Laut visuell darzustellen.

Die Intensitätsplots wurden mit Peptid- und Intensitätskarten sowie mit einer visuellen Inspektion der Mikro-Array Scans korreliert, um Peptide und Konsensthemen zu identifizieren, die mit den Plasmaproben interagierten.

Dort, wo es unklar war, ob eine gewisse Aminosäure zur Bindung von Antikörpern beitrug, wurden die entsprechenden Buchstaben in grauer Farbe geschrieben.

### Beispiel 5: Peptidkartierung von CENPA

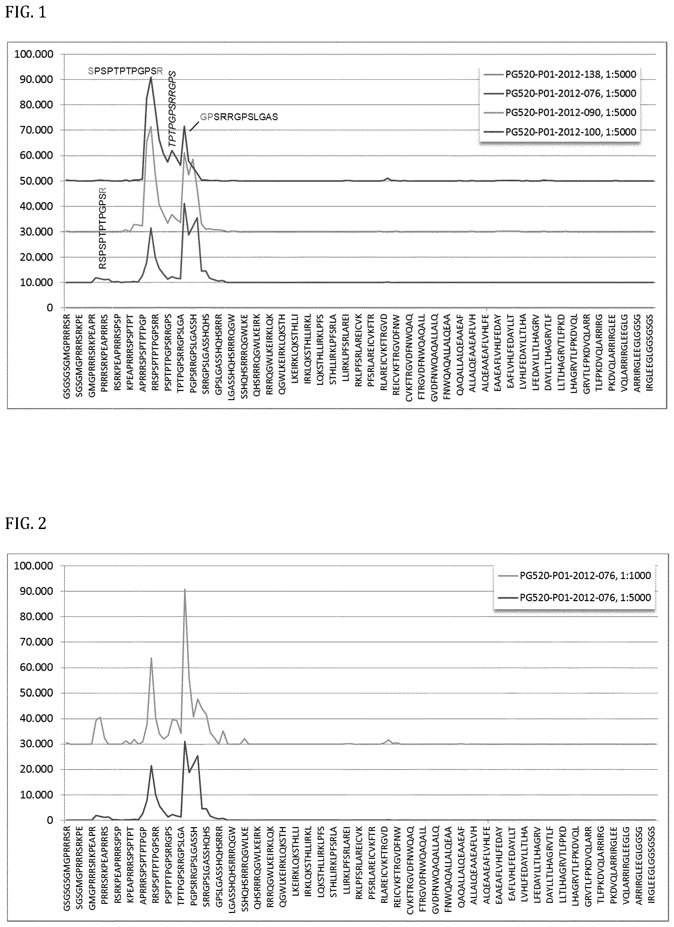

Wie in Figur 1 dargestellt, unterstrichen die Intensitätsplots (Verdünnung 1:5000) der CENPA Peptide die sehr starke und fast identische Reaktion der Seren PG520-P01-2012-076, PG520-P01-2012-090 und PG520-P01-2012-100 mit den beiden hauptsächlichen Epitopen RSPSPTPTPGPSR (SEQ ID No. 13) und GPSRRGPSLGAS (SEQ ID No. 11) und einem dritten, jedoch überlagerten (überlappenden) Epitop TPTPGPSRRGPS (SEQ ID No. 12).

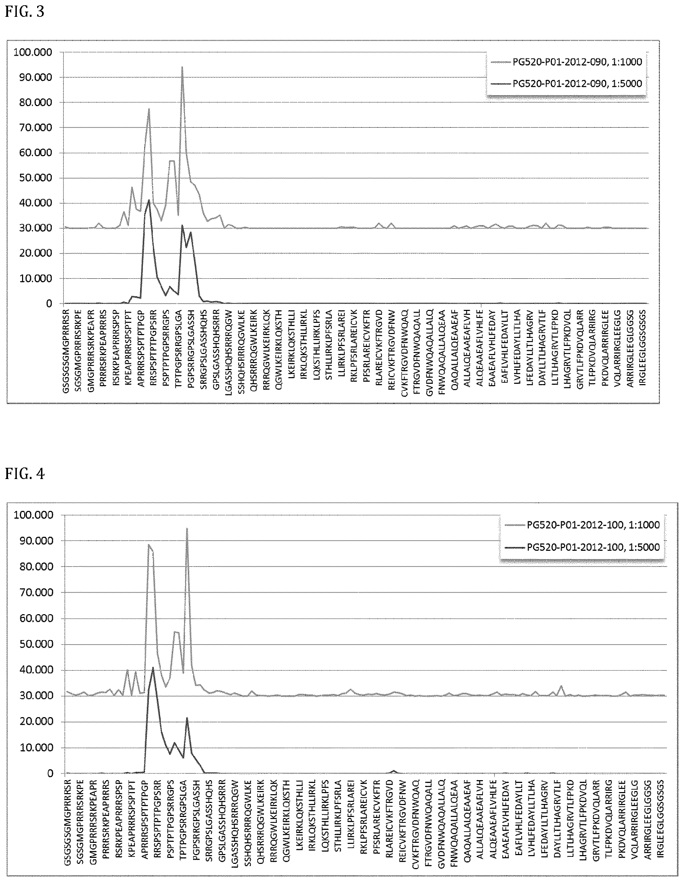

Alle diese drei einzelnen Peptide sind durch die Aminosäuren (aa) 16-36 (Figur 1) repräsentiert. Das Serum PG520-P01-2012-076 zeigte einen leichten Unterschied in der Intensitätsratio von beiden Haupt-Epitopen und verursachte ein zusätzliches N-Terminus Epitop RSPSPTPTPGPSR (SEQ ID No. 15) bzw. PRRRSRKPEAPR (SEQ ID No. 14)(repräsentiert durch aa 3-14). Dieses Epitop reagiert auch schwach in der Probe PG520-P01-2012-090 bei einer Verdünnung von 1:1000 auf (Figur 3). Darüber hinaus zeigt die Probe PG520-P01-2012-100 eine zusätzliche Reaktion auf diese N-Terminus Region bei einer Verdünnung von 1:1000 (Figur 4).

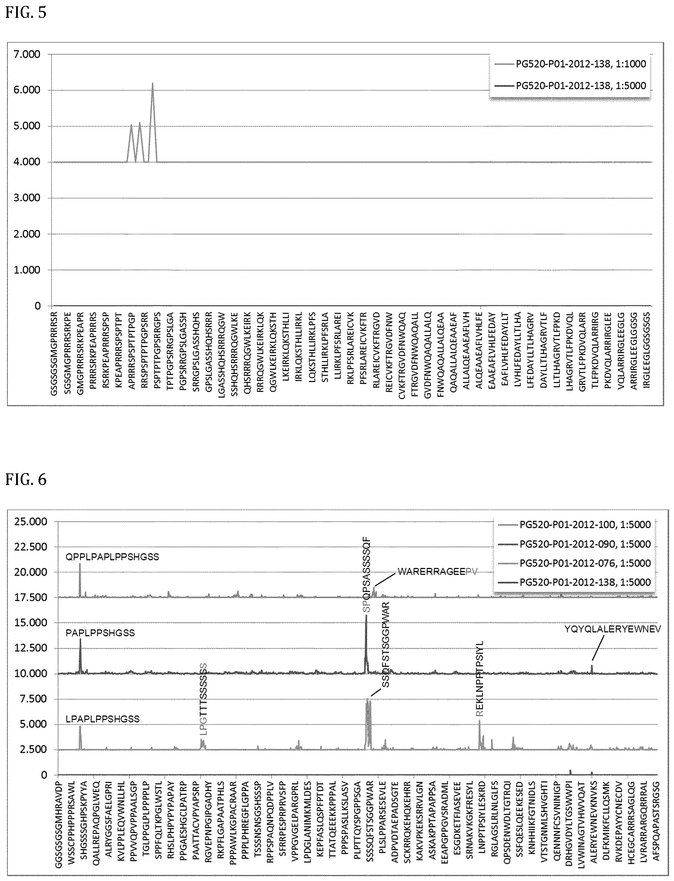

Die Patientenprobe PG520-P01-2012-138, die in dem Luminex Perl-Assay negativ für anti-CENPA getestet wurde, zeigt eine schwache Reaktion (Figur 5). Es wurden lediglich drei einzelne Peptid Interaktionen auf der Basis der Peptide APRRRSPSPTPTPGP (SEQ ID No. 16), RRRSPSPTPTPGPSR (SEQ ID No. 17) und SPSPTPTPGPSRRGP (SEQ ID No. 18) bei einer Verdünnung von 1:1000 beobachtet, jedoch nicht bei einer Verdünnung von 1:5000.

### Beispiel 6: Die Verteilung der Epitope von CENPA

Die Verteilung der Epitope, welche mittels Peptidkartierung identifiziert wurden, ist in Tabelle 2 dargestellt.

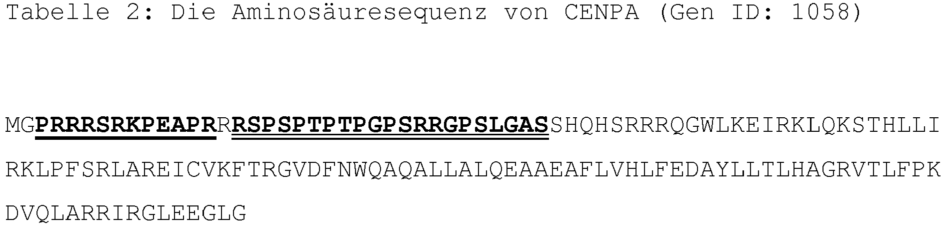

Das größere durch Peptidkartierung identifizierte Epitop ist doppelt unterstrichen (aa 16-36), während das zweite, schwächere Epitop einfach unterstrichen ist (aa 3-14).

Muro et al. haben eine Serie abgeschnittener Peptide in E. coli exprimiert und mit 91 ACA-positiven Seren eine Immunblot Analyse durchgeführt (Muro et. al., 2000). Achtzig der Seren (88%) mit ACA reagierten auf die N-Terminus Region mit 52 Aminosäuren, während keine der Seren auf den C-Terminus reagierten. Zwei synthetische Peptide (Aminosäuresequenzen aa 3±17 (Peptid A) und aa 25±38 (Peptid B)) reagierten in ELISA auf 78 (86%) und 79 (87%) der ACA-positiven Seren. Peptid A entspricht dem zweiten Epitop (aa 3-14), welches mit unseren gegenwärtig getesteten Patientenproben weniger reaktiv war als von Muro et. al (2000) beschrieben. Peptid B ist ein Teil des größeren durch Peptid Arrays identifizierten Epitop (aa 16-36) .

Mithilfe des systematischen Ansatzes von Peptidkartierung können nicht nur einzelne Epitope in der Größenordnung der gegenwärtig eingesetzten Peptide (14 aa), sondern auch größere Regionen, die von multiplen, überlappenden Peptiden bedeckt sind, identifiziert werden.

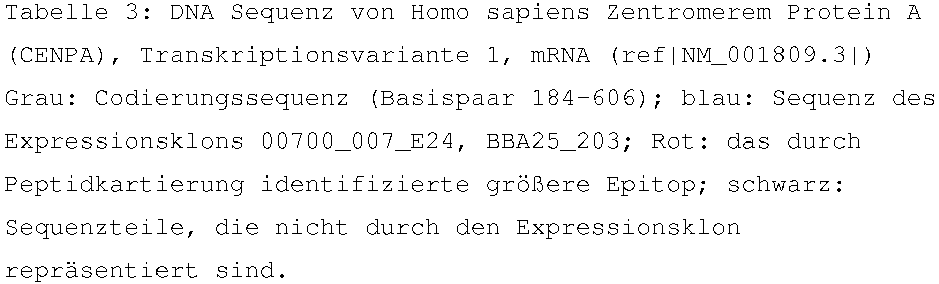

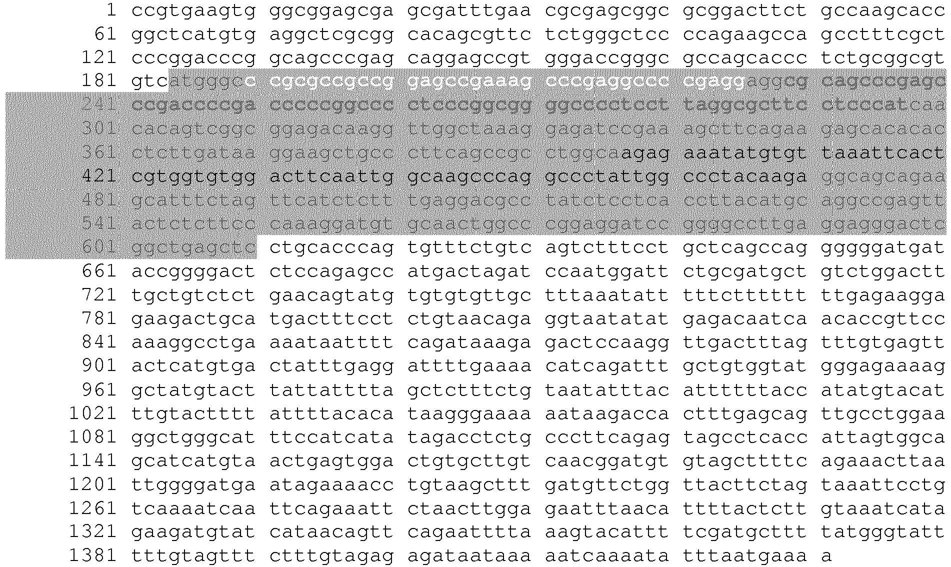

### Beispiel 6: Peptidkartierung von KDM6B

Wie in Figur 6 dargestellt, unterstrichen die Intensitätsplots (Verdünnung 1:5000) die moderat bis starke und teilweise ähnliche Reaktion der Seren PG520-P01-2012-076, PG520-P01-2012-090 und PG520-P01-2012-100 mit den Haupt-Epitopen LPAPLPPSHGSS (SEQ ID No. 2) und SPQPSASSSSQF (SEQ ID No. 3). Das LPAPLPPSHGSS Epitop (SEQ ID No. 2) (aa 55-67) war allen positiven Serumproben gemein.

Im Gegensatz dazu, wurde das SPQPSASSSSQF ((SEQ ID No. 3) aa 861-873) Epitop nur in den Seren PG520-P01-2012-076 und PG520-P01-2012-090 beobachtet. Das benachbarte Epitop SSQFSTSGGPWAR (SEQ ID No. 4) (aa 870-881) wurde jedoch ebenfalls in Probe PG520-P01-2012-076 und GGPWARERRAGEEPV (SEQ ID No. 6)(aa 877-890) erkannt und wurde geringfügig erkannt durch PG520-P01-2012-100 (1:1000 Verdünnung). Das Resultat bedeutet eine immunogene Region von aa 861 bis aa 890.

Andere Epitope, wie beispielsweise REKLNPPTPSIYL (SEQ ID No. 5) wurden nur in einer der Proben identifiziert.

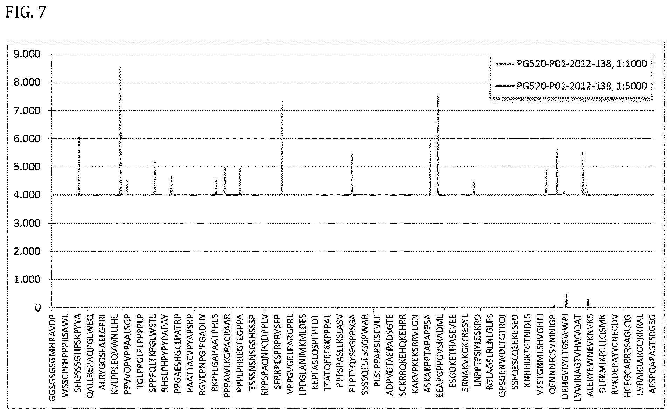

Die Patientenprobe PG520-P01-2012-138, die im Luminex Perl-Assay negativ für KDM6B testete, zeigt eine schwache bis moderate Reaktion auf einige einzelne Peptide bei einer Verdünnung von 1:1000, sowie auf zwei verschiedene Peptide bei einer Verdünnung von 1:5000. Die meisten Interaktionen basierten entweder auf hoch alkalischen Peptiden wie beispielsweise KRNYGAKRGGPPVKR (SEQ ID No. 19) oder hydrophoben Peptiden mit einem Prolin an dem C-Terminus (z.B. FPKTPEVGPGPPPGP (SEQ ID No. 20) oder GHPSKPYYAPGAPTP (SEQ ID No. 21). Aufgrund der verschwommenen Morphologie der Spots, der schwachen Spotintensitäten und der allgemeinen Widersprüchlichkeit der Daten wurden alle Interaktionen als nicht spezifisch eingestuft (Figur 7).

### Beispiel 7: Verteilung der Epitope von KDM6B und Repräsentation von KDM6B in Sero-Tag

Die Verteilung der durch Peptidkartierung identifizierten Epitope ist in Tabelle 4 dargestellt. In den Sero-Tag Discovery Screens exprimieren drei Klonen KDM6B Antigen (bzw. Fragmente von KDM6B). Alle drei Expressionsklonen exprimieren eine ähnliche Codierungsregion (aa 42-435) und umfassen das größere LPAPLPPSHGSS (SEQ ID No. 2) Epitop.

Eine zweite immunogene Region, die von > 3 Peptiden (aa 861-890) bedeckt wird, wird nicht von diesen Expressionsklonen repräsentiert. Die Kombination von beiden immunogenen Epitopen / Region wird die Entdeckung KDM6B-positiver Proben verbessern.

Die Epitopkartierung von KDM6B ergibt zwei größere Epitope und nur ein Epitop, das in einem von drei KDM6B-positiven Proben erkannt wurde. Alle drei verfügbaren Expressionsklonen von KDM6B exprimieren die ähnliche Koderegion (aa 42-435) und umfassen das eine der größeren Epitope (LPAPLPPSHGSS, SEQ ID No. 2). Die zweite immunogene Region, die von > 3 Peptiden (aa 861-890) bedeckt ist, ist nicht durch diese Expressionsklonen repräsentiert.

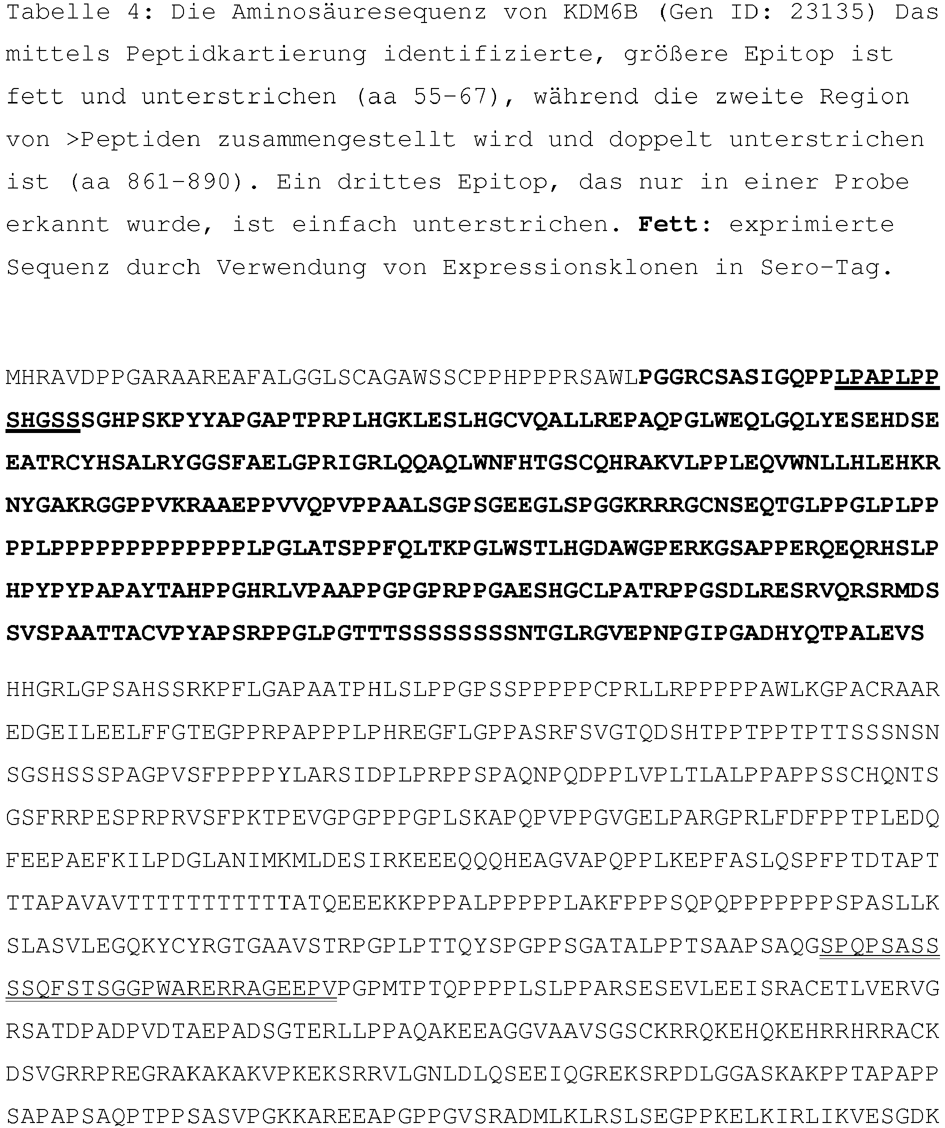

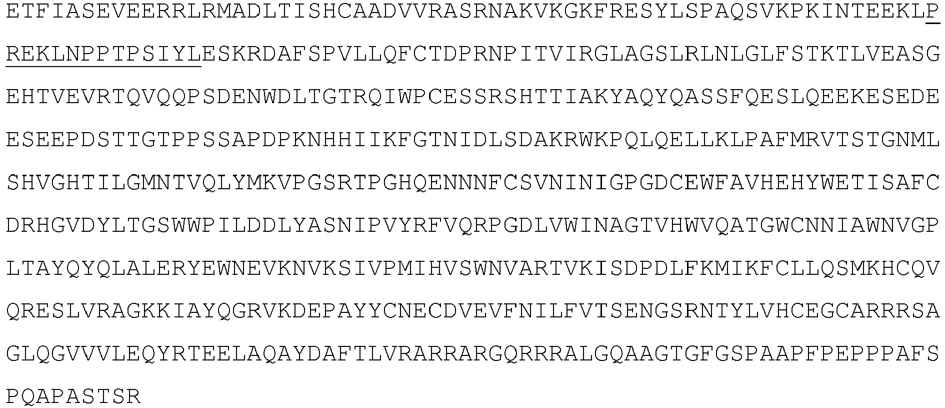

### Literatur:

Mehra S, Walker J, Patterson K, Fritzler MJ (2013). Autoantibodies in systemic sclerosis. Autoimmun Rev. 12(3) :340-54.
Mierau R, Moinzadeh P, Riemekasten G, Melchers I, Meurer M, Reichenberger F, Buslau M, Worm M, Blank N, Hein R, Müller-Ladner U, Kuhn A, Sunderkötter C, Juche A, Pfeiffer C, Fiehn C, Sticherling M, Lehmann P, Stadler R, Schulze-Lohoff E, Seitz C, Foeldvari I, Krieg T, Genth E, Hunzelmann N (2011). Frequency of disease-associated and other nuclear autoantibodies in patients of the German Network for Systemic Scleroderma: correlation with characteristic clinical features. Arthritis Res Ther. 13(5):R172
LeRoy EC, Black C, Fleischmajer R, Jablonska S, Krieg T, Medsger TA Jr, Rowell N, Wollheim F (1988). Scleroderma (systemic sclerosis): classification, subsets and pathogenesis. J Rheumatol. 15(2):202-5.
Watts R., (2006). Autoantibodies in the autoimmune rheumatic diseases, Medicine, 34 (11): 441-444

## Patentansprüche

1. Verwendung von einem Marker zum in vitro oder ex vivo Nachweis von Systemische Sklerose (SSc), **dadurch gekennzeichnet, dass** der Marker ein Peptid ist, welches aus einer Sequenz ausgewählt aus
| | |
|---|---|
| SEQ ID No. 1 | SPQPSASSSSQFSTSGGPWARERRAGEEPV, |
| SEQ ID No. 3 | SPQPSASSSSQF, |
| SEQ ID No. 4 | SSQFSTSGGPWAR, |
| SEQ ID No. 6 | GGPWARERRAGEEPV, |
oder einer Teilsequenz aus SEQ ID No: 1 besteht,
wobei die Teilsequenz eine Länge von 12 oder 13 Aminosäuren hat.

2. Verwendung nach dem Anspruch 1, **dadurch gekennzeichnet, dass** der Marker in Kombination mit einem oder mehreren weitern Marker verwendet wird, wobei der eine oder mehrere Marker unabhänging voneinander ausgewählt ist aus einem weiteren Peptid, welches aus einer Sequenz ausgewählt aus SEQ ID NOs: 1-18 besteht.

3. Verwendung nach einem der Ansprüche 1 oder 2, **dadurch gekennzeichnet, dass** der Marker ein Peptid ist, welches aus einer Sequenz ausgewählt aus SEQ ID No. 1, SEQ ID No. 3, SEQ ID No. 4, oder SEQ ID No. 6 besteht.

4. Verwendung nach einem der Ansprüche 1-3, **dadurch gekennzeichnet, dass** der Marker ein Peptid ist, welches aus SEQ ID No. 1 besteht.

5. Verwendung nach einem der Ansprüche 1-3, **dadurch gekennzeichnet, dass** der Marker ein Peptid ist, welches aus SEQ ID No. 3 besteht.

6. Verwendung nach einem der Ansprüche 1-3, **dadurch gekennzeichnet, dass** der Marker ein Peptid ist, welches aus SEQ ID No. 4 besteht.

7. Verwendung nach einem der Ansprüche 1-3, **dadurch gekennzeichnet, dass** der Marker ein Peptid ist, welches aus SEQ ID No. 6 besteht.

8. Verwendung nach einem der Ansprüche 1-7 zur Diagnose von SSc, und/oder zur Therapieüberwachung bei SSc.

9. Ein fester Träger auf dem ein oder mehrere Marker fixiert sind, wobei der eine oder mehrere Marker ein Peptid ist, welches aus einer Sequenz ausgewählt aus SEQ ID No. 1 , SEQ ID No. 3, SEQ ID No. 4, oder SEQ ID No. 6 oder einer Teilsequenz aus SEQ ID No: 1 besteht,
wobei die Teilsequenz eine Länge von 12 oder 13 Aminosäuren hat.

## Claims

1. Use of a marker for in-vitro or ex-vivo detection of systemic sclerosis (SSc), **characterised in that** the marker is a peptide, which consists of a sequence selected from
SEQ ID no. 1 SPQPSASSSSQFSTSGGPWARERRAGEEPV,
SEQ ID no. 3 SPQPSASSSSQF,
SEQ ID no. 4 SSQFSTSGGPWAR,
SEQ ID no. 6 GGPWARERRAGEEPV,
or a part sequence of SEQ ID no. 1, wherein the part sequence has a length of 12 or 13 amino acids.

2. Use according to claim 1, **characterised in that** the marker is used in combination with one or more further markers, wherein the one or more markers is selected independently from each other from a further peptide, which consists of a sequence selected from SEQ ID nos. 1-18.

3. Use according to one of the claims 1 or 2, **characterised in that** the marker is a peptide, which consists of a sequence selected from SEQ ID no. 1, SEQ ID no. 3, SEQ ID no. 4 or SEQ ID no. 6.

4. Use according to one of the claims 1-3, **characterised in that** the marker is a peptide, which consists of SEQ ID no. 1.

5. Use according to one of the claims 1-3, **characterised in that** the marker is a peptide, which consists of SEQ ID no. 3.

6. Use according to one of the claims 1-3, **characterised in that** the marker is a peptide, which consists of SEQ ID no. 4.

7. Use according to one of the claims 1-3, **characterised in that** the marker is a peptide, which consists of SEQ ID no. 6.

8. Use according to one of the claims 1-7 for the diagnosis of SSc and/or for therapy monitoring of SSc.

9. A solid substrate, on which one or more markers are fixed, wherein the one or more markers is a peptide, which consists of a sequence selected from SEQ ID no. 1, SEQ ID no. 3, SEQ ID no. 4 or SEQ ID no. 6 or a part sequence of SEQ ID no. 1, wherein the part sequence has a length of 12 or 13 amino acids.

## Revendications

1. Utilisation d'un marqueur pour la détection in vitro ou ex vivo d'une sclérose systémique (SSc), **caractérisée en ce que** le marqueur est un peptide qui est choisi parmi l'une des séquences suivantes
| | |
|---|---|
| SEQ ID No. 1 | SPQPSASSSSQFSTSGGPWARERRAGEEPV |
| SEQ ID No. 3 | SPQPSASSSSQF |
| SEQ ID No. 4 | SSQFSTSGGPWAR |
| SEQ ID No. 6 | GGPWARERRAGEEPV |
ou est constitué d'une séquence partielle de SEQ ID No. 1,
la séquence partielle ayant une longueur de 12 ou 13 acides aminés.

2. Utilisation selon la revendication 1, **caractérisée en ce que** le marqueur est utilisé en combinaison avec un ou plusieurs autres marqueurs,
le ou les plusieurs marqueurs étant choisis indépendamment les uns des autres parmi un autre peptide, qui est constitué d'une séquence choisie parmi SEQ ID No. 1 à 18.

3. Utilisation selon l'une des revendications 1 ou 2, **caractérisée en ce que** le marqueur est un peptide qui est constitué d'une séquence choisie parmi SEQ ID No. 1, SEQ ID No. 3, SEQ ID No. 4 ou SEQ ID No. 6.

4. Utilisation selon l'une des revendications 1 à 3, **caractérisée en ce que** le marqueur est un peptide qui est constitué de SEQ ID No. 1.

5. Utilisation selon l'une des revendications 1 à 3, **caractérisée en ce que** le marqueur est un peptide qui est constitué de SEQ ID No. 3.

6. Utilisation selon l'une des revendications 1 à 3, **caractérisée en ce que** le marqueur est un peptide qui est constitué de SEQ ID No. 4.

7. Utilisation selon l'une des revendications 1 à 3, **caractérisée en ce que** le marqueur est un peptide qui est constitué de SEQ ID No. 6.

8. Utilisation selon l'une des revendications 1 à 7, pour le diagnostic de la SSc et/ou pour la surveillance de la thérapie d'une SSc.

9. Support solide sur lequel sont fixés un ou plusieurs marqueurs, le ou les plusieurs marqueurs étant des peptides qui sont constitués d'une séquence choisie parmi SEQ ID No. 1, SEQ ID No. 3, SEQ ID No. 4 ou SEQ ID No. 6, ou d'une séquence partielle de SEQ ID No. 1,
la séquence partielle ayant une longueur de 12 ou 13 acides aminés.


### SEQUENCE LISTING

<110> Protagen AG
<120> PRO150WO_SSc
<130> PRO150WO
<140> 00
   <141> 2015-07-04
<160> 120
<170> PatentIn version 3.3
<210> 1
   <211> 30
   <212> PRT
   <213> Homo sapiens
<400> 1
   Ser Pro Gln Pro Ser Ala Ser Ser Ser Ser Gln Phe Ser Thr Ser Gly
   1               5                   10                  15
   Gly Pro Trp Ala Arg Glu Arg Arg Ala Gly Glu Glu Pro Val
               20                  25                  30
<210> 2
   <211> 12
   <212> PRT
   <213> Homo sapiens
<400> 2
   Leu Pro Ala Pro Leu Pro Pro Ser His Gly Ser Ser
   1               5                   10
<210> 3
   <211> 12
   <212> PRT
   <213> Homo sapiens
<400> 3
   Ser Pro Gln Pro Ser Ala Ser Ser Ser Ser Gln Phe
   1               5                   10
<210> 4
   <211> 13
   <212> PRT
   <213> Homo sapiens
<400> 4
   Ser Ser Gln Phe Ser Thr Ser Gly Gly Pro Trp Ala Arg
   1               5                   10
<210> 5
   <211> 13
   <212> PRT
   <213> Homo sapiens
<400> 5
   Arg Glu Lys Leu Asn Pro Pro Thr Pro Ser Ile Tyr Leu
   1               5                   10
<210> 6
   <211> 15
   <212> PRT
   <213> Homo sapiens
<400> 6
   Gly Gly Pro Trp Ala Arg Glu Arg Arg Ala Gly Glu Glu Pro Val
   1               5                   10                  15
<210> 7
   <211> 14
   <212> PRT
   <213> Homo sapiens
<400> 7
   Pro Arg Glu Lys Leu Asn Pro Pro Thr Pro Ser Ile Tyr Leu
   1               5                   10
<210> 8
   <211> 15
   <212> PRT
   <213> Homo sapiens
<400> 8
   Tyr Gln Tyr Gln Leu Ala Leu Glu Arg Tyr Glu Trp Asn Glu Val
   1               5                   10                  15
<210> 9
   <211> 34
   <212> PRT
   <213> Homo sapiens
<400> 9
   Pro Arg Arg Arg Ser Arg Lys Pro Glu Ala Pro Arg Arg Arg Ser Pro
   1               5                   10                  15
   Ser Pro Thr Pro Thr Pro Gly Pro Ser Arg Arg Gly Pro Ser Leu Gly
               20                  25                  30
   Ala Ser
<210> 10
   <211> 12
   <212> PRT
   <213> Homo sapiens
<400> 10
   Ser Pro Ser Pro Thr Pro Thr Pro Gly Pro Ser Arg
   1               5                   10
<210> 11
   <211> 12
   <212> PRT
   <213> Homo sapiens
<400> 11
   Gly Pro Ser Arg Arg Gly Pro Ser Leu Gly Ala Ser
   1               5                   10
<210> 12
   <211> 12
   <212> PRT
   <213> Homo sapiens
<400> 12
   Thr Pro Thr Pro Gly Pro Ser Arg Arg Gly Pro Ser
   1               5                   10
<210> 13
   <211> 13
   <212> PRT
   <213> Homo sapiens
<400> 13
   Arg Ser Pro Ser Pro Thr Pro Thr Pro Gly Pro Ser Arg
   1               5                   10
<210> 14
   <211> 12
   <212> PRT
   <213> Homo sapiens
<400> 14
   Pro Arg Arg Arg Ser Arg Lys Pro Glu Ala Pro Arg
   1               5                   10
<210> 15
   <211> 13
   <212> PRT
   <213> Homo sapiens
<400> 15
   Arg Ser Pro Ser Pro Thr Pro Thr Pro Gly Pro Ser Arg
         1               5                   10
<210> 16
   <211> 15
   <212> PRT
   <213> Homo sapiens
<400> 16
   Ala Pro Arg Arg Arg Ser Pro Ser Pro Thr Pro Thr Pro Gly Pro
   1               5                   10                  15
<210> 17
   <211> 15
   <212> PRT
   <213> Homo sapiens
<400> 17
   Arg Arg Arg Ser Pro Ser Pro Thr Pro Thr Pro Gly Pro Ser Arg
   1 5 10 15
<210> 18
   <211> 15
   <212> PRT
   <213> Homo sapiens
<400> 18
   Ser Pro Ser Pro Thr Pro Thr Pro Gly Pro Ser Arg Arg Gly Pro
   1 5 10 15
<210> 19
   <211> 15
   <212> PRT
   <213> Homo sapiens
<400> 19
   Lys Arg Asn Tyr Gly Ala Lys Arg Gly Gly Pro Pro Val Lys Arg
   1 5 10 15
<210> 20
   <211> 15
   <212> PRT
   <213> Homo sapiens
<400> 20
   Phe Pro Lys Thr Pro Glu Val Gly Pro Gly Pro Pro Pro Gly Pro
   1 5 10 15
<210> 21
   <211> 15
   <212> PRT
   <213> Homo sapiens
<400> 21
   Gly His Pro Ser Lys Pro Tyr Tyr Ala Pro Gly Ala Pro Thr Pro
   1 5 10 15
<210> 22
   <211> 140
   <212> PRT
   <213> Homo sapiens
<400> 22
   Met Gly Pro Arg Arg Arg Ser Arg Lys Pro Glu Ala Pro Arg Arg Arg
   1               5                   10                  15
   Ser Pro Ser Pro Thr Pro Thr Pro Gly Pro Ser Arg Arg Gly Pro Ser
               20                  25                  30
   Leu Gly Ala Ser Ser His Gln His Ser Arg Arg Arg Gln Gly Trp Leu
           35                  40                  45
   Lys Glu Ile Arg Lys Leu Gln Lys Ser Thr His Leu Leu Ile Arg Lys
       50                  55                  60
   Leu Pro Phe Ser Arg Leu Ala Arg Glu Ile Cys Val Lys Phe Thr Arg
   65                  70                  75                  80
   Gly Val Asp Phe Asn Trp Gln Ala Gln Ala Leu Leu Ala Leu Gln Glu
                   85                  90                  95
   Ala Ala Glu Ala Phe Leu Val His Leu Phe Glu Asp Ala Tyr Leu Leu
               100                 105                 110
   Thr Leu His Ala Gly Arg Val Thr Leu Phe Pro Lys Asp Val Gln Leu
           115                 120                 125
   Ala Arg Arg Ile Arg Gly Leu Glu Glu Gly Leu Gly
       130                 135                 140
<210> 23
   <211> 1431
   <212> DNA
   <213> Homo sapiens
<400> 23
   ccgtgaagtg ggcggagcga gcgatttgaa cgcgagcggc gcggacttct gccaagcacc      60
   ggctcatgtg aggctcgcgg cacagcgttc tctgggctcc ccagaagcca gcctttcgct     120
   cccggacccg gcagcccgag caggagccgt gggaccgggc gccagcaccc tctgcggcgt     180
   gtcatgggcc cgcgccgccg gagccgaaag cccgaggccc cgaggaggcg cagcccgagc     240
   ccgaccccga cccccggccc ctcccggcgg ggcccctcct taggcgcttc ctcccatcaa     300
   cacagtcggc ggagacaagg ttggctaaag gagatccgaa agcttcagaa gagcacacac     360
   ctcttgataa ggaagctgcc cttcagccgc ctggcaagag aaatatgtgt taaattcact     420
   cgtggtgtgg acttcaattg gcaagcccag gccctattgg ccctacaaga ggcagcagaa     480
   gcatttctag ttcatctctt tgaggacgcc tatctcctca ccttacatgc aggccgagtt     540
   actctcttcc caaaggatgt gcaactggcc cggaggatcc ggggccttga ggagggactc     600
   ggctgagctc ctgcacccag tgtttctgtc agtctttcct gctcagccag gggggatgat     660
   accggggact ctccagagcc atgactagat ccaatggatt ctgcgatgct gtctggactt     720
   tgctgtctct gaacagtatg tgtgtgttgc tttaaatatt tttctttttt ttgagaagga     780
   gaagactgca tgactttcct ctgtaacaga ggtaatatat gagacaatca acaccgttcc     840
   aaaggcctga aaataatttt cagataaaga gactccaagg ttgactttag tttgtgagtt     900
   actcatgtga ctatttgagg attttgaaaa catcagattt gctgtggtat gggagaaaag     960
   gctatgtact tattatttta gctctttctg taatatttac attttttacc atatgtacat   1020
   ttgtactttt attttacaca taagggaaaa aataagacca ctttgagcag ttgcctggaa   1080
   ggctgggcat ttccatcata tagacctctg cccttcagag tagcctcacc attagtggca   1140
   gcatcatgta actgagtgga ctgtgcttgt caacggatgt gtagcttttc agaaacttaa   1200
   ttggggatga atagaaaacc tgtaagcttt gatgttctgg ttacttctag taaattcctg   1260
   tcaaaatcaa ttcagaaatt ctaacttgga gaatttaaca ttttactctt gtaaatcata   1320
   gaagatgtat cataacagtt cagaatttta aagtacattt tcgatgcttt tatgggtatt   1380
   tttgtagttt ctttgtagag agataataaa aatcaaaata tttaatgaaa a             1431
<210> 24
   <211> 1682
   <212> PRT
   <213> Homo sapiens
<400> 24
<210> 25
   <211> 15
   <212> PRT
   <213> Homo sapiens
<400> 25
   Gly Ser Gly Ser Gly Ser Gly Met Gly Pro Arg Arg Arg Ser Arg
   1 5 10 15
<210> 26
   <211> 15
   <212> PRT
   <213> Homo sapiens
<400> 26
   Ser Gly Ser Gly Met Gly Pro Arg Arg Arg Ser Arg Lys Pro Glu
   1 5 10 15
<210> 27
   <211> 15
   <212> PRT
   <213> Homo sapiens
<400> 27
   Gly Met Gly Pro Arg Arg Arg Ser Arg Lys Pro Glu Ala Pro Arg
   1 5 10 15
<210> 28
   <211> 15
   <212> PRT
   <213> Homo sapiens
<400> 28
   Pro Arg Arg Arg Ser Arg Lys Pro Glu Ala Pro Arg Arg Arg Ser
   1 5 10 15
<210> 29
   <211> 15
   <212> PRT
   <213> Homo sapiens
<400> 29
   Arg Ser Arg Lys Pro Glu Ala Pro Arg Arg Arg Ser Pro Ser Pro
   1 5 10 15
<210> 30
   <211> 15
   <212> PRT
   <213> Homo sapiens
<400> 30
   Lys Pro Glu Ala Pro Arg Arg Arg Ser Pro Ser Pro Thr Pro Thr
   1 5 10 15
<210> 31
   <211> 15
   <212> PRT
   <213> Homo sapiens
<400> 31
   Ala Pro Arg Arg Arg Ser Pro Ser Pro Thr Pro Thr Pro Gly Pro
   1 5 10 15
<210> 32
   <211> 15
   <212> PRT
   <213> Homo sapiens
<400> 32
   Arg Arg Ser Pro Ser Pro Thr Pro Thr Pro Gly Pro Ser Arg Arg
   1 5 10 15
<210> 33
   <211> 15
   <212> PRT
   <213> Homo sapiens
<400> 33
   Pro Ser Pro Thr Pro Thr Pro Gly Pro Ser Arg Arg Gly Pro Ser
   1               5                   10                  15
<210> 34
   <211> 15
   <212> PRT
   <213> Homo sapiens
<400> 34
   Thr Pro Thr Pro Gly Pro Ser Arg Arg Gly Pro Ser Leu Gly Ala
   1               5                   10                  15
<210> 35
   <211> 15
   <212> PRT
   <213> Homo sapiens
<400> 35
   Pro Gly Pro Ser Arg Arg Gly Pro Ser Leu Gly Ala Ser Ser His
   1               5                   10                  15
<210> 36
   <211> 15
   <212> PRT
   <213> Homo sapiens
<400> 36
   Ser Arg Arg Gly Pro Ser Leu Gly Ala Ser Ser His Gln His Ser
   1               5                   10                  15
<210> 37
   <211> 15
   <212> PRT
   <213> Homo sapiens
<400> 37
   Gly Pro Ser Leu Gly Ala Ser Ser His Gln His Ser Arg Arg Arg
   1               5                   10                  15
<210> 38
   <211> 15
   <212> PRT
   <213> Homo sapiens
<400> 38
   Leu Gly Ala Ser Ser His Gln His Ser Arg Arg Arg Gln Gly Trp
   1 5 10 15
<210> 39
   <211> 15
   <212> PRT
   <213> Homo sapiens
<400> 39
   Ser Ser His Gln His Ser Arg Arg Arg Gln Gly Trp Leu Lys Glu
   1 5 10 15
<210> 40
   <211> 15
   <212> PRT
   <213> Homo sapiens
<400> 40
   Gln His Ser Arg Arg Arg Gln Gly Trp Leu Lys Glu Ile Arg Lys
   1 5 10 15
<210> 41
   <211> 15
   <212> PRT
   <213> Homo sapiens
<400> 41
   Arg Arg Arg Gln Gly Trp Leu Lys Glu Ile Arg Lys Leu Gln Lys
   1 5 10 15
<210> 42
   <211> 15
   <212> PRT
   <213> Homo sapiens
<400> 42
   Gln Gly Trp Leu Lys Glu Ile Arg Lys Leu Gln Lys Ser Thr His
   1 5 10 15
<210> 43
   <211> 15
   <212> PRT
   <213> Homo sapiens
<400> 43
   Leu Lys Glu Ile Arg Lys Leu Gln Lys Ser Thr His Leu Leu Ile
   1 5 10 15
<210> 44
   <211> 15
   <212> PRT
   <213> Homo sapiens
<400> 44
   Ile Arg Lys Leu Gln Lys Ser Thr His Leu Leu Ile Arg Lys Leu
   1               5                   10                  15
<210> 45
   <211> 15
   <212> PRT
   <213> Homo sapiens
<400> 45
   Leu Gln Lys Ser Thr His Leu Leu Ile Arg Lys Leu Pro Phe Ser
   1               5                   10                  15
<210> 46
   <211> 15
   <212> PRT
   <213> Homo sapiens
<400> 46
   Ser Thr His Leu Leu Ile Arg Lys Leu Pro Phe Ser Arg Leu Ala
   1               5                   10                  15
<210> 47
   <211> 15
   <212> PRT
   <213> Homo sapiens
<400> 47
   Leu Leu Ile Arg Lys Leu Pro Phe Ser Arg Leu Ala Arg Glu Ile
   1               5                   10                  15
<210> 48
   <211> 15
   <212> PRT
   <213> Homo sapiens
<400> 48
   Arg Lys Leu Pro Phe Ser Arg Leu Ala Arg Glu Ile Cys Val Lys
   1               5                   10                  15
<210> 49
   <211> 15
   <212> PRT
   <213> Homo sapiens
<400> 49
   Pro Phe Ser Arg Leu Ala Arg Glu Ile Cys Val Lys Phe Thr Arg
   1               5                   10                  15
<210> 50
   <211> 15
   <212> PRT
   <213> Homo sapiens
<400> 50
   Arg Leu Ala Arg Glu Ile Cys Val Lys Phe Thr Arg Gly Val Asp
   1 5 10 15
<210> 51
   <211> 15
   <212> PRT
   <213> Homo sapiens
<400> 51
   Arg Glu Ile Cys Val Lys Phe Thr Arg Gly Val Asp Phe Asn Trp
   1 5 10 15
<210> 52
   <211> 15
   <212> PRT
   <213> Homo sapiens
<400> 52
   Cys Val Lys Phe Thr Arg Gly Val Asp Phe Asn Trp Gln Ala Gln
   1 5 10 15
<210> 53
   <211> 15
   <212> PRT
   <213> Homo sapiens
<400> 53
   Phe Thr Arg Gly Val Asp Phe Asn Trp Gln Ala Gln Ala Leu Leu
   1 5 10 15
<210> 54
   <211> 15
   <212> PRT
   <213> Homo sapiens
<400> 54
   Gly Val Asp Phe Asn Trp Gln Ala Gln Ala Leu Leu Ala Leu Gln
   1 5 10 15
<210> 55
   <211> 15
   <212> PRT
   <213> Homo sapiens
<400> 55
   Phe Asn Trp Gln Ala Gln Ala Leu Leu Ala Leu Gln Glu Ala Ala
   1 5 10 15
<210> 56
   <211> 15
   <212> PRT
   <213> Homo sapiens
<400> 56
   Gln Ala Gln Ala Leu Leu Ala Leu Gln Glu Ala Ala Glu Ala Phe
   1 5 10 15
<210> 57
   <211> 15
   <212> PRT
   <213> Homo sapiens
<400> 57
   Ala Leu Leu Ala Leu Gln Glu Ala Ala Glu Ala Phe Leu Val His
   1 5 10 15
<210> 58
   <211> 15
   <212> PRT
   <213> Homo sapiens
<400> 58
   Ala Leu Gln Glu Ala Ala Glu Ala Phe Leu Val His Leu Phe Glu
   1 5 10 15
<210> 59
   <211> 15
   <212> PRT
   <213> Homo sapiens
<400> 59
   Glu Ala Ala Glu Ala Phe Leu Val His Leu Phe Glu Asp Ala Tyr
   1 5 10 15
<210> 60
   <211> 15
   <212> PRT
   <213> Homo sapiens
<400> 60
   Glu Ala Phe Leu Val His Leu Phe Glu Asp Ala Tyr Leu Leu Thr
   1 5 10 15
<210> 61
   <211> 15
   <212> PRT
   <213> Homo sapiens
<400> 61
   Leu Val His Leu Phe Glu Asp Ala Tyr Leu Leu Thr Leu His Ala
   1 5 10 15
<210> 62
   <211> 15
   <212> PRT
   <213> Homo sapiens
<400> 62
   Leu Phe Glu Asp Ala Tyr Leu Leu Thr Leu His Ala Gly Arg Val
   1               5                   10                  15
<210> 63
   <211> 15
   <212> PRT
   <213> Homo sapiens
<400> 63
   Asp Ala Tyr Leu Leu Thr Leu His Ala Gly Arg Val Thr Leu Phe
   1               5                   10                  15
<210> 64
   <211> 15
   <212> PRT
   <213> Homo sapiens
<400> 64
   Leu Leu Thr Leu His Ala Gly Arg Val Thr Leu Phe Pro Lys Asp
   1               5                   10                  15
<210> 65
   <211> 15
   <212> PRT
   <213> Homo sapiens
<400> 65
   Leu His Ala Gly Arg Val Thr Leu Phe Pro Lys Asp Val Gln Leu
   1               5                   10                  15
<210> 66
   <211> 15
   <212> PRT
   <213> Homo sapiens
<400> 66
   Gly Arg Val Thr Leu Phe Pro Lys Asp Val Gln Leu Ala Arg Arg
   1               5                   10                  15
<210> 67
   <211> 15
   <212> PRT
   <213> Homo sapiens
<400> 67
   Thr Leu Phe Pro Lys Asp Val Gln Leu Ala Arg Arg Ile Arg Gly
   1 5 10 15
<210> 68
   <211> 15
   <212> PRT
   <213> Homo sapiens
<400> 68
   Pro Lys Asp Val Gln Leu Ala Arg Arg Ile Arg Gly Leu Glu Glu
   1 5 10 15
<210> 69
   <211> 15
   <212> PRT
   <213> Homo sapiens
<400> 69
   Val Gln Leu Ala Arg Arg Ile Arg Gly Leu Glu Glu Gly Leu Gly
   1 5 10 15
<210> 70
   <211> 15
   <212> PRT
   <213> Homo sapiens
<400> 70
   Ala Arg Arg Ile Arg Gly Leu Glu Glu Gly Leu Gly Gly Ser Gly
   1 5 10 15
<210> 71
   <211> 15
   <212> PRT
   <213> Homo sapiens
<400> 71
   Ile Arg Gly Leu Glu Glu Gly Leu Gly Gly Ser Gly Ser Gly Ser
   1 5 10 15
<210> 72
   <211> 15
   <212> PRT
   <213> Homo sapiens
<400> 72
   Gly Gly Ser Gly Ser Gly Ser Gly Met His Arg Ala Val Asp Pro
   1 5 10 15
<210> 73
   <211> 15
   <212> PRT
   <213> Homo sapiens
<400> 73
   Trp Ser Ser Cys Pro Pro His Pro Pro Pro Arg Ser Ala Trp Leu
   1               5                   10                  15
<210> 74
   <211> 15
   <212> PRT
   <213> Homo sapiens
<400> 74
   Ser His Gly Ser Ser Ser Gly His Pro Ser Lys Pro Tyr Tyr Ala
   1               5                   10                  15
<210> 75
   <211> 15
   <212> PRT
   <213> Homo sapiens
<400> 75
   Gln Ala Leu Leu Arg Glu Pro Ala Gln Pro Gly Leu Trp Glu Gln
   1               5                   10                  15
<210> 76
   <211> 15
   <212> PRT
   <213> Homo sapiens
<400> 76
   Ala Leu Arg Tyr Gly Gly Ser Phe Ala Glu Leu Gly Pro Arg Ile
   1               5                   10                  15
<210> 77
   <211> 15
   <212> PRT
   <213> Homo sapiens
<400> 77
   Lys Val Leu Pro Pro Leu Glu Gln Val Trp Asn Leu Leu His Leu
   1               5                   10                  15
<210> 78
   <211> 15
   <212> PRT
   <213> Homo sapiens
<400> 78
   Pro Pro Val Val Gln Pro Val Pro Pro Ala Ala Leu Ser Gly Pro
   1               5                   10                  15
<210> 79
   <211> 15
   <212> PRT
   <213> Homo sapiens
<400> 79
   Thr Gly Leu Pro Pro Gly Leu Pro Leu Pro Pro Pro Pro Leu Pro
   1               5                   10                  15
<210> 80
   <211> 15
   <212> PRT
   <213> Homo sapiens
<400> 80
   Ser Pro Pro Phe Gln Leu Thr Lys Pro Gly Leu Trp Ser Thr Leu
   1               5                   10                  15
<210> 81
   <211> 15
   <212> PRT
   <213> Homo sapiens
<400> 81
   Arg His Ser Leu Pro His Pro Tyr Pro Tyr Pro Ala Pro Ala Tyr
   1               5                   10                  15
<210> 82
   <211> 15
   <212> PRT
   <213> Homo sapiens
<400> 82
   Pro Pro Gly Ala Glu Ser His Gly Cys Leu Pro Ala Thr Arg Pro
   1               5                   10                  15
<210> 83
   <211> 15
   <212> PRT
   <213> Homo sapiens
<400> 83
   Pro Ala Ala Thr Thr Ala Cys Val Pro Tyr Ala Pro Ser Arg Pro
   1               5                   10                  15
<210> 84
   <211> 15
   <212> PRT
   <213> Homo sapiens
<400> 84
   Arg Gly Val Glu Pro Asn Pro Gly Ile Pro Gly Ala Asp His Tyr
   1 5 10 15
<210> 85
   <211> 15
   <212> PRT
   <213> Homo sapiens
<400> 85
   Arg Lys Pro Phe Leu Gly Ala Pro Ala Ala Thr Pro His Leu Ser
   1 5 10 15
<210> 86
   <211> 15
   <212> PRT
   <213> Homo sapiens
<400> 86
   Pro Pro Pro Ala Trp Leu Lys Gly Pro Ala Cys Arg Ala Ala Arg
   1 5 10 15
<210> 87
   <211> 15
   <212> PRT
   <213> Homo sapiens
<400> 87
   Pro Pro Pro Leu Pro His Arg Glu Gly Phe Leu Gly Pro Pro Ala
   1 5 10 15
<210> 88
   <211> 15
   <212> PRT
   <213> Homo sapiens
<400> 88
   Thr Ser Ser Ser Met Ser Met Ser Gly Ser His Ser Ser Ser Pro
   1 5 10 15
<210> 89
   <211> 15
   <212> PRT
   <213> Homo sapiens
<400> 89
   Arg Pro Pro Ser Pro Ala Gln Asn Pro Gln Asp Pro Pro Leu Val
   1 5 10 15
<210> 90
   <211> 15
   <212> PRT
   <213> Homo sapiens
<400> 90
   Ser Phe Arg Arg Pro Glu Ser Pro Arg Pro Arg Val Ser Phe Pro
   1               5                   10                  15
<210> 91
   <211> 15
   <212> PRT
   <213> Homo sapiens
<400> 91
   Val Pro Pro Gly Val Gly Glu Leu Pro Ala Arg Gly Pro Arg Leu
   1               5                   10                  15
<210> 92
   <211> 15
   <212> PRT
   <213> Homo sapiens
<400> 92
   Leu Pro Asp Gly Leu Ala Asn Ile Met Lys Met Leu Asp Glu Ser
   1               5                   10                  15
<210> 93
   <211> 15
   <212> PRT
   <213> Homo sapiens
<400> 93
   Lys Glu Pro Phe Ala Ser Leu Gln Ser Pro Phe Pro Thr Asp Thr
   1               5                   10                  15
<210> 94
   <211> 15
   <212> PRT
   <213> Homo sapiens
<400> 94
   Thr Thr Ala Thr Gln Glu Glu Glu Lys Lys Pro Pro Pro Ala Leu
   1               5                   10                  15
<210> 95
   <211> 15
   <212> PRT
   <213> Homo sapiens
<400> 95
   Pro Pro Pro Ser Pro Ala Ser Leu Leu Lys Ser Leu Ala Ser Val
   1               5                   10                  15
<210> 96
   <211> 15
   <212> PRT
   <213> Homo sapiens
<400> 96
   Pro Leu Pro Thr Thr Gln Tyr Ser Pro Gly Pro Pro Ser Gly Ala
   1               5                   10                  15
<210> 97
   <211> 15
   <212> PRT
   <213> Homo sapiens
<400> 97
   Ser Ser Ser Ser Gln Phe Ser Thr Ser Gly Gly Pro Trp Ala Arg
   1               5                   10                  15
<210> 98
   <211> 15
   <212> PRT
   <213> Homo sapiens
<400> 98
   Pro Leu Ser Leu Pro Pro Ala Arg Ser Glu Ser Glu Val Leu Glu
   1               5                   10                  15
<210> 99
   <211> 15
   <212> PRT
   <213> Homo sapiens
<400> 99
   Ala Asp Pro Val Asp Thr Ala Glu Pro Ala Asp Ser Gly Thr Glu
   1               5                   10                  15
<210> 100
   <211> 15
   <212> PRT
   <213> Homo sapiens
<400> 100
   Ser Cys Lys Arg Arg Gln Lys Glu His Gln Lys Glu His Arg Arg
   1               5                   10                  15
<210> 101
   <211> 15
   <212> PRT
   <213> Homo sapiens
<400> 101
   Lys Ala Lys Val Pro Lys Glu Lys Ser Arg Arg Val Leu Gly Asn
   1               5                   10                  15
<210> 102
   <211> 15
   <212> PRT
   <213> Homo sapiens
<400> 102
   Ala Ser Lys Ala Lys Pro Pro Thr Ala Pro Ala Pro Pro Ser Ala
   1 5 10 15
<210> 103
   <211> 15
   <212> PRT
   <213> Homo sapiens
<400> 103
   Glu Glu Ala Pro Glu Pro Pro Gly Val Ser Arg Ala Asp Met Leu
   1 5 10 15
<210> 104
   <211> 15
   <212> PRT
   <213> Homo sapiens
<400> 104
   Glu Ser Gly Asp Lys Glu Thr Phe Ile Ala Ser Glu Val Glu Glu
   1 5 10 15
<210> 105
   <211> 15
   <212> PRT
   <213> Homo sapiens
<400> 105
   Ser Arg Asn Ala Lys Val Lys Gly Lys Phe Arg Glu Ser Tyr Leu
   1 5 10 15
<210> 106
   <211> 15
   <212> PRT
   <213> Homo sapiens
<400> 106
   Leu Asn Pro Pro Thr Pro Ser Ile Tyr Leu Glu Ser Lys Arg Asp
   1 5 10 15
<210> 107
   <211> 15
   <212> PRT
   <213> Homo sapiens
<400> 107
   Arg Gly Leu Ala Gly Ser Leu Arg Leu Asn Leu Gly Leu Phe Ser
   1 5 10 15
<210> 108
   <211> 15
   <212> PRT
   <213> Homo sapiens
<400> 108
   Gln Pro Ser Asp Glu Asn Trp Asp Leu Thr Gly Thr Arg Gln Ile
   1 5 10 15
<210> 109
   <211> 15
   <212> PRT
   <213> Homo sapiens
<400> 109
   Ser Ser Phe Gln Glu Ser Leu Gln Glu Glu Lys Glu Ser Glu Asp
   1 5 10 15
<210> 110
   <211> 15
   <212> PRT
   <213> Homo sapiens
<400> 110
   Lys Asn His His Ile Ile Lys Phe Gly Thr Asn Ile Asp Leu Ser
   1 5 10 15
<210> 111
   <211> 15
   <212> PRT
   <213> Homo sapiens
<400> 111
   Val Thr Ser Thr Gly Asn Met Leu Ser His Val Gly His Thr Ile
   1 5 10 15
<210> 112
   <211> 15
   <212> PRT
   <213> Homo sapiens
<400> 112
   Gln Glu Asn Asn Asn Phe Cys Ser Val Asn Ile Asn Ile Gly Pro
   1 5 10 15
<210> 113
   <211> 15
   <212> PRT
   <213> Homo sapiens
<400> 113
   Asp Arg His Gly Val Asp Tyr Leu Thr Gly Ser Trp Trp Pro Ile
   1 5 10 15
<210> 114
   <211> 15
   <212> PRT
   <213> Homo sapiens
<400> 114
   Leu Val Trp Ile Asn Ala Gly Thr Val His Trp Val Gln Ala Thr
   1 5 10 15
<210> 115
   <211> 15
   <212> PRT
   <213> Homo sapiens
<400> 115
   Ala Leu Glu Arg Tyr Glu Trp Asn Glu Val Lys Asn Val Lys Ser
   1 5 10 15
<210> 116
   <211> 15
   <212> PRT
   <213> Homo sapiens
<400> 116
   Asp Leu Phe Lys Met Ile Lys Phe Cys Leu Leu Gln Ser Met Lys
   1 5 10 15
<210> 117
   <211> 15
   <212> PRT
   <213> Homo sapiens
<400> 117
   Arg Val Lys Asp Glu Pro Ala Tyr Tyr Cys Asn Glu Cys Asp Val
   1 5 10 15
<210> 118
   <211> 15
   <212> PRT
   <213> Homo sapiens
<400> 118
   His Cys Glu Gly Cys Ala Arg Arg Arg Ser Ala Gly Leu Gln Gly
   1 5 10 15
<210> 119
   <211> 15
   <212> PRT
   <213> Homo sapiens
<400> 119
   Leu Val Arg Ala Arg Arg Ala Arg Gly Gln Arg Arg Arg Ala Leu
   1               5                   10                  15
<210> 120
   <211> 15
   <212> PRT
   <213> Homo sapiens
<400> 120
   Ala Phe Ser Pro Gln Ala Pro Ala Ser Thr Ser Arg Gly Ser Gly
   1               5                   10                  15